(19) Europäisches Patentamt
European Patent Office
Office européen des brevets

(11) Publication number: **0 147 175**
**A2**

# (12) EUROPEAN PATENT APPLICATION

(21) Application number: 84308923.6

(22) Date of filing: 19.12.84

(51) Int.Cl.⁴: C 12 P 21/02
C 12 N 15/00, C 07 K 15/26
A 61 K 45/02

(30) Priority: 20.12.83 JP 241457/83

(43) Date of publication of application:
03.07.85 Bulletin 85/27

(84) Designated Contracting States:
AT BE CH DE FR GB IT LI LU NL SE

(71) Applicant: SUNTORY KABUSHIKI KAISHA, also known as SUNTORY LTD.
1-40, Dojimahama 2-chome
Kita-ku Osaka530(JP)

(72) Inventor: Okada, Tsutomu
38-2 Nishigoshonouchi-cho Kinugasa Kita-ku
Kyoto 603(JP)

(72) Inventor: Yamamoto, Kazumori
5-18-104 Wakayamadai 1-chome Shimamoto-cho
Mishima-gun Osaka 618(JP)

(72) Inventor: Tanaka, Shoji
8-46 Minamikaneden-cho 1-chome Suita
Osaka 564(JP)

(72) Inventor: Oshima, Takehiro
17-10-383 Bessho-hommachi Takatsuki
Osaka 569(JP)

(74) Representative: Burford, Anthony Frederick
W.H. Beck, Greener & Co. 7 Stone Buildings Lincoln's Inn
London WC2A 3SZ(GB)

## (54) Novel basic polypeptides and a method of producing thereof.

(57) A novel basic polypeptide having an immune interferon-like activity consists of 131 or 132 amino acid residues and has an apparent molecular weight of about 16,000 as measured by SDS electrophresis and of about 17,000 as measured by the high performance liquid chromatographic molecular sieve method; an isoelectric point (PI value) in the range of 9.2-10.0; a cystein or methionine residue as the amino terminal; a lysine residue as the carboxy terminal; and the amino acid composition: Asx 20, Thr 5, Ser 9, Glx 16, Pro 2, Gly 4, Ala 7, Cys 2, Val 8, Met 3 or 4, Ile 7, Leu 9, Tyr 5, Phe 9, Lys 19, His 2, Arg 3 and Trp 1 (Asx including Asp and Asn, and Glx including Glu and Gln). The polypeptide is produced by cultivating a microorganism such as *Escherichia coli* transformed with a plasmid containing a chemically synthesized DNA sequence coding for the corresponding amino acid sequence and extracting the polypeptide from the culture.

EP 0 147 175 A2

# Novel basic polypeptides and a method of producing thereof

This invention relates to novel basic polypeptides and, more specifically, to novel, human gamma-type interferon-like polypeptides and a method of producing the same.

According to their physico-chemical and immunological properties as well as differing cells and inducers for their production, human interferons are classified into three groups, namely α, β and γ types. All the three types of interferons are each a substance comprising a polypeptide having potent antiviral activity (naturally occurring β-type and γ-type ones are said to be polypeptides having a sugar chain, namely glycoproteins). In particular, γ-type interferons (also called immune interferons) have stronger antiproliferative activity as compared with α-type and β-type interferons and therefore are expected to be useful not only as antiviral agents but also as antitumor agents or immunomodulating agents.

Structural analysis of the corresponding gene has already shown that human γ-type interferon (hereinafter referred to as hIFN-γ) is a polypeptide consisting of 146 amino acids as formed from its precursor containing a signal peptide composed of 20 amino acid residues through appropriate processing (Devos, R. et al., Nucl.

Acids Res., 10: 2487, 1982 and Gray, P. W. et al., Nature, 295: 503, 1982). Furthermore, patent applications have been laid open disclosing methods of producing pre-hIFN-γ (signal peptide-containing hIFN-γ precursor) and hIFN-γ (mature-type hIFN-γ) by the recombinant DNA technology (Japanese Kokai Tokkyo Koho Nos. 90514/1983, 174396/1983 and 189197/1983).

However, many of physico-chemical properties of γ-type interferons, including natural hIFN-γ (glycoprotein) inducedly produced by human lymphocytes or human spleen cells, remain unclarified. Moreover, γ-type interferons are said to be difficult to purify because they are physico-chemically unstable as compared with α-type and β-type interferons (hereinafter referred to as IFN-α and IFN-β, respectively).

To clear these problems, the present inventors conducted intensive studies in search of physico-chemically stable hIFN-γ analogs retaining high physiological activity and easily producible and purifiable and found that a basic polypeptide having the properties specifically given in Claim 1 or a polypeptide composed of 131 or 132 amino acid residues as shown in Claim 2 (hereinafter referred to as GIF131) is a substance meeting the above requirements. The present invention has been completed based on the above finding.

Although the structure of natural hIFN-γ comprising 146 amino acid residues as presented by Devos et al.

(Nucl. Acids Res., 10: 2487, 1982) or Gray et al. (Nature, 295: 503, 1982) includes the amino acid sequence of the polypeptide provided by the present invention, the superior physico-chemical and physiological properties of the polypeptide according to the invention are by no means obvious from the above-mentioned natural hIFN-γ (hereinafter referred to as GIF146), and the substance according to the invention can be said to be a distinctly new polypeptide having potent immune-interferon activity.

The purified polypeptide of the invention has 1) an apparent molecular weight of about 16,000 (16 K) as determined by SDS electrophoresis using a polyacrylamide gel and an apparent molecular weight of about 17,000 (17 K) as determined by the high performance liquid chromatographic molecular sieve method; 2) an isoelectric point (PI value) of about 9.3 or 9.8 as determined by the chromatographic focusing method or the plate method, respectively; 3) an amino acid composition as given in Table 1; 4) elemental analysis data as shown in Table 2; 5) an ultraviolet absorption spectrum as shown in Fig. 12; 6) a circular dichroism curve as shown in Fig. 13; 7) a nuclear magnetic resonance (NMR) spectrum as shown in Fig. 14; and 8) an infrared absorption spectrum as shown in Fig. 15. Amino acid sequence analysis, the details of which are to be described later, has determined partially the sequence of said polypeptide and also confirmed that the amino terminal is Met or Cys and the carboxyl terminal is Lys.

The amino acid composition data and the results of elemental analysis and other physico-chemical analyses have demonstrated that the substance according to the invention is composed of 131 or 132 amino acid residues and is a polypeptide having the amino acid sequence as described in Claim 2.

Furthermore, it has also been shown that the polypeptide according to the invention is differing in physico-chemical properties from natural hIFN-γ (GIF146) and is a pharmaceutically useful substance having potent immune interferon activity, as shown in the example. The polypeptide according to the invention is readily soluble in water and buffers in common use and can remain relatively stable, with respect to its physiological activity, during treatment in SDS or at low pH. Therefore, the polypeptide can easily be extracted from the culture of a microorganism capable of producing the same and can be purified easily as well. It is further expected that said polypeptide can readily be made up into pharmaceutical dosage form preparations such as antitumor, anticancer, antiviral and immunomodulating preparations. It is also expected that potentiated therapeutic effects can be produced by combined use of the polypeptide according to the invention with other pharmaceutical substances such as other types of interferons, TNF (tumor necrosis factor), IL-2 (interleukin 2), CBF (cancer breaking factor), MIF (macrophage inhibitory factor), CSF (colony stimulating factor) and other lymphokines.

The polypeptide according to the invention can be obtained from the culture of a microorganism transformed with an approrpiate expression vector which allows the expression of the corresponding DNA gene inserted therein and comprising a DNA sequence coding for the amino acid sequence described in Claim 2, 3 or 22, for example the chemically synthesized DNA sequence as described in Claim 14. Surprisingly, said polypeptide could also be obtained by extraction, followed by purification, from the culture of a microorganism transformed with an appropriate expression vector with a DNA sequence coding for the amino acid sequence described in Claim 4 or 20, such as the DNA sequence described in Claim 22, inserted therein, for example the Escherichia coli transformant described in Claim 16 or 17 (W3110/pIN5GIF54, WA802/pIN5GIF54, W3110/pYtacGIF or WA802/pYtacGIF). The Escherichia coli transformants described in Claim 16 and Claim 17 are disclosed in Japanese Patent Applications Nos. 132524/1983 and 162337/1983, respectively.

The DNA gene coding for the polypeptide according to the invention can be obtained, as described in Japanese Patent Application No. 86180/1982, by chemically synthesizing DNA fragments each having an appropriate length by the phosphotriester method and ligating the DNA fragments together. Said DNA gene can also be obtained, as mentioned hereinbelow, from the DNA sequence (chemically synthesized DNA gene coding for GIF146) by application of the in vitro or site directed mutation method using the so-called M13 phage (Zoller, M. J. & Smith, M., Nucl. Acids Res., 10: 6487, 1982). Thus, for instance:

1) A GIF146-encoding gene (having an EcoRI site at the 5' end, with the SalI site at the 3' end filled in using T4 polymerase) is inserted into the EcoRI-HincII fragment of the M13 mp 9 phage [from Bethesda Research Laboratories Inc. (hereinafter, BRL)] dsDNA (double-stranded DNA), and the product is used for transformation of E. coli K12-derived JM103 (from BRL). White plaque-forming colonies on YT medium (0.8% tryptone, 0.5% yeast extract, 0.5% sodium chloride, 1.5% agar) containing X-gal and IPTG are chosen, followed by phage DNA (hereinafter, RFDNA) extraction and checking, based on the DNA size, restriction enzyme cleavage sites and base sequence, among others, whether the isolate is the desired transformant.

2) Then, said phage is separated from the transformant culture supernatant, followed by extraction of the phage DNA (single-stranded DNA: ssDNA) therefrom. This DNA is annealed with a chemically synthesized primer DNA fragment such as a single-stranded DNA fragment comprising the DNA sequence ($^{5'}$AAAACTGGTAAG$^{3'}$) coding for the amino acid sequence covering from the 128th through 131st amino acid of the polypeptide described in Claim 5 or 20 and, directly downstream therefrom, the translation termination codon (TAA) and the cleavage recognition sequence for the restriction enzyme SalI (GTCGAC) and, further downstream therefrom, the DNA sequence ($^{5'}$TCTCAGATGCTG$^{3'}$) coding for the amino acid sequence covering from the 135th through 138th amino acid of the polypeptide described in Claim 5 or 20, connected in series.

3) *E. coli* DNA polymerase I large fragment (Klenow fragment) and T4 ligase, together with dNTP (standing for dATP, dTTP, dGTP and dCTP collectively), are added to the above-mentioned annealed DNA solution to thereby effect the DNA synthesis reaction. In this manner, there is obtained a dsDNA with partial mismatching (for the chemically synthesized DNA sequence, in the portion of TAAGTCGAC and, for the phage DNA, in the partial DNA sequence CGTAAAAGA coding for the amino acid sequence covering from the 132nd through 134th amino acid of the above-mentioned polypeptide).

4) JM103 (already mentioned) is transformed with the above dsDNA and grown on YT medium. Each white plaque-forming colony is cultured mixedly with JM103, and the DNA (RFDNA) is extracted and analyzed using restriction enzymes, to give the desired colony. Said phage is then used for infection of JM103, followed by allowing plaque formation again. Adequate plaque selection and the subsequent mixed cultivation with JM103 are followed by DNA (RFDNA) extraction. Whether the DNA has the desired sequence, namely the DNA sequence described in Claim 14, or not is checked by analysis using restriction enzymes and determination of the DNA sequence, among others. The DNA fragment having the desired DNA sequence is inserted, for example into the plasmid pIN5GIF54 or ptacGIF (described in Japanese Patent Application No. 132524/1983 or 162337/1983, respectively) between the EcoRI-SalI cleavage sites, whereby a plasmid vector capable of producing the polypeptide of the invention in an efficient manner can be obtained.

Referring to the accompanying drawings, Fig. 1 shows the results of DEAE cellulose DE52 chromatography of the extract; Fig. 2 shows the results of CM Sephadex C-50 chromatography of the IFN-active fractions obtained in DE52 chromatography; Fig. 3 shows the results of CM cellulose C-52 chromatography of the IFN-active fractions (fractions Nos. 20 through 30) obtained in C-50 chromatography. Fig. 4 outlines the cloning of the GIF146 gene fragment into M13 mp 9 RFDNA; Fig. 5 outlines the method of preparing RFDNA containing the GIF131 gene by *in vitro* mutation; Fig. 6 outlines the construction of the GIF131 gene expression vector pIN5GIF54-131. Fig. 7 shows the elution pattern in high performance liquid chromatography of the polypeptide purified in the practice of the invention. Fig. 8-a shows the polyacrylamide SDS electrophoretic patterns for the polypeptide according to the invention and the GIF146 polypeptide as well as standard proteins. A is for the GIF146 polypeptide, B, D and F are for standard proteins, C and E are for the polypeptide according to the invention, and G is for a mixture of GIF146 and the polypeptide according to the invention. Fig. 8-b is a graphic representation of the distances of travel in electrophoresis of the above polypeptides and standard proteins. Fig. 9 is a graphic representation of the elution patterns for the polypeptide according to the invention and standard proteins in molecular sieve high performance liquid chromatography. Fig. 10 shows the results of chromatography for the determination of the isoelectric point (PI value) of the polypeptide according to the invention as performed by the isoelectrofocusing method. Fig. 11 shows the amino acid sequence of the polypeptide according to the invention as composed of 131 amino acid residues and the primary structure analyzed (arrows). Fig. 12 through Fig. 15 show the UV absorption spectrum, circular dichroism, NMR spectrum and infrared absorption spectrum of the polypeptide according to the invention, respectively.

In the following example, a method of producing the novel basic polypeptide according to the invention is described. However, the scope of the invention is by no means limited thereby.

Example

I. Production of novel human gamma-type interferon-like polypeptide, (1)

1. Extraction from Escherichia coli transformant culture

Two liters of M9 medium for Escherichia coli containing casamino acids (3%) was inoculated with the Escherichia coli transformant W3110/pIN5GIF54 (inoculum size being 1% of the medium). Incubation was carried out under aeration and stirring at 30°C for 24 hours, and cells were harvested by centrifugation (8,000 rpm, 10 minutes). The 2-liter culture gave about 40 g of wet cells. About 130 g of wet cells obtained by three 2-liter cultures were suspended in 1 liter of 50 mM phosphate buffer, pH 6.0 (hereinafter, KPB) and then collected by centrifugation and again suspended in 1 liter of KPB. After cooling the suspension to 10°C or below, the Escherichia coli cells were disrupted using a Manton Gaulin homogenizer model 15M.

The homogenate was then centrifuged (7,000 rpm, 20 minutes), giving a precipitate and a supernatant. Most of the interferon (IFN) activity was found in the supernatant, the potency being $3.9 \times 10^5$ U/ml. (The method of potency determination is mentioned later.)

The supernatant (about 1 liter) was adjusted to pH 7.0 with 1 N sodium hydroxide and, after addition of 243 g of ammonium sulfate per liter with cooling (5°C to 10°C), allowed to stand for 30 minutes to 1 hour, followed by centrifugation (7,000 rpm, 20 minutes) to give a precipitate and a supernatant. Most of the IFN activity was found in the supernatant. To this supernatant (about 1 liter), there was added 285 g per liter of ammonium sulfate with cooling (5°C to 10°C), and the mixture was allowed to stand for 30 minutes to 1 hour, followed by centrifugation (7,000 rpm, 20 minutes) to give again a precipitate and a supernatant. Most of the IFN activity was found in the precipitate. The precipitate was collected and dissolved in a small amount of ice-cooled 20 mM Tris-hydrochloric acid buffer, pH 7.5 (hereinafter, THB). The amount of THB should preferably be one tenth of the liquid volume before salting out with ammonium sulfate.

The solution was placed in a dialyzing tube (Union Carbide) and dialyzed against about 20 volumes of 10 mM THB (pH 7.5) in a cold room (about 5°C) for more than 3 hours. This dialysis procedure was repeated twice more each time following exchange of the dialyzing fluid for a fresh portion. After dialysis, the dialyzate solution was centrifuged (10,000 rpm, 20 minutes) under cooling to thereby remove the precipitate. Most of the IFN activity was found in the supernatant and the specific activity (IFN activity based on the protein amount) was about 8 x $10^5$ U/mg protein.

2. Purification by ion exchange resin chromatography

a. DEAE cellulose chromatography

DEAE cellulose DE52 (Whatman) was washed well with distilled water and suspended in 20 mM THB (pH 7.5), and the suspension was adjusted to pH 7.5 with 1 N hydrochloric acid and then packed into a column in an amount of 1 ml per 20 mg of protein contained in the above dialyzate solution. The above dialyzate was applied to this column at a flow rate of about 0.2 ml/$cm^2$. Then, the column was washed with about 3 volumes (on the dialyzate basis) of 20 mM THB (pH 7.5). The effluents (dialyzate effluent and washing THB effluent) were fractionated in 10-ml portions and each fraction was assayed for protein content based on the absorption at 280 nm (A280) and by the Folin method, and for IFN activity (cf. Fig. 1). The dialyzate effluent fraction (in Fig. 1, fractions Nos. 10-30) had a specific activity of about 1 x $10^6$ U/mg protein.

b. CM Sephadex C-50 chromatography

CM Sephadex C-50 (Pharmacia) was washed well with distilled water to attain sufficient swelling, then suspended in 20 mM THB (pH 7.5) and packed into a column in an amount of 1 ml per 5 mg of protein contained in the DE52 column effluent. The effluent from the DE52 column was applied to this column to thereby cause adsorption of the desired polypeptide thereon. The column was washed with an equal volume of 20 mM THB (pH 7.5), followed by elution with about 5 volumes (on the column volume basis) of 20 mM THB (pH 7.5) containing NaCl with a concetration gradient from 50 mM NaCl to 300 mM NaCl. The pattern of elution of the desired polypeptide is shown in Fig. 2. The IFN activity was found in fractions eluted at an NaCl concentration of about 100 mM and the peak specific activity was about 5 x $10^6$ U/mg protein.

c. CM cellulose chromatography

CM cellulose C-52 (Whatman) was washed well with distilled water and further washed with 500 mM ammonium formate for equilibration. Then, it was washed well with 10 mM ammonium formate and packed into a column in an amount of 1 ml per 2 mg of protein. The IFN-active eluate fractions (in Fig. 2, fractions Nos. 20 through 30) from Sephadex C-50 chromatography were diluted with 5 volumes of distilled water and applied to the above column to thereby cause adsorption of the desired polypeptide thereon. The column was then washed with an equal volume of 10 mM ammonium formate and then eluted with about 5 volumes (on the column volume basis) of aqueous ammonium formate with a concentration gradient from 10 mM to 200 mM. The pattern of elution of the desired polypeptide is shown in Fig. 3. Main IFN-active fractions were found at an ammonium formate concentration of about 110 mM to 130 mM (in Fig. 3, peak III) and the specific activity was 1 x $10^7$ U/mg protein.

The above C-52 chromatography gave 4 peaks of proteins (I, II, III and IV) (cf. Fig. 3). However, polyacrylamide SDS electrophoresis revealed that the desired IFN-active polypeptide (polypeptide composed of 131 or 132 amino acid residues) was present in the fractions corresponding to peak III (fractions Nos 65 through 70).

II. Production of novel human gamma-type interferon-like polypeptide, (2)

An example of a method of producing the polypeptide (GIF131) of the invention from *Escherichia coli* transformed with a plasmid containing DNA gene coding for the polypeptide of Claim 2 is described below.

1. Construction of GIF131-encoding gene

a. Insertion of GIF146 gene into M13 DNA (cf. Fig. 4) In 30 µl of TA buffer (33 mM Tris-hydrochloric acid buffer, pH 7.6, containing 66 mM potassium acetate, 10 mM magnesium acetate and 0.5 mM dithiothreitol), 0.5 µg of M13mp9 RFDNA (BRL) was digested by warming with *Eco*RI and *Hinc*II (1 unit each) at 37°C for 1 hour, and a straight chain DNA having an *Eco*RI cohesive end at one end and a *Hinc*II terminus (flush end) at the other was separated by 1% agarose gel electrophoresis.

Separately, a GIF131-encoding DNA was obtained, as mentioned below, from the plasmid pIN5GIF54 (described in Japanese Patent Application No. 132524/1983) containing a GIF146-encoding DNA by *in vitro* mutation.

The pIN5GIF54 DNA (5 µg) was dissolved in 50 µl of TA buffer and, following addition of *Sal*I (30 units), the solution was warmed at 37°C for 1 hour. Then, 1 µl of dNTP solution (solution containing dATP, dCTP, dGTP and dTTP each in a concentration of 25 mM) and T4 DNA polymerase (1 unit) were added, and the mixture was warmed at 37°C for 45 minutes to thereby fill in the cohesive end resulting from *Sal*I cleavage. The enzymatic reaction was terminated by heating at 65°C for 30 minutes. EcoRI (10 units) was then added and the reaction was allowed to proceed at 37°C for 1 hour. Following the subsequent 1.5% agarose gel electrophoresis, a gel portion corresponding to an about 450 bp DNA fragment was excised and said DNA fragment was extracted and purified by the electro-elute method.

The above two DNA fragments were reacted with each other in 20 µl of ligation solution (20 mM Tris-hydrochloric acid solution, pH 7.5, containing 10 mM magnesium chloride, 1 mM ATP and 10 mM dithiothreitol) in the presence of 5 units of T4 DNA ligase (Boehringer Mannheim) at 14°C for 18 hours. A 3-µl portion of the reaction mixture was added to a suspension in 50 mM calcium chloride of Escherichia coli JM103 (purchased from BRL) treated with calcium chloride in the conventional manner, to thereby effect transformation. Transformant clone selection was performed in the following manner.

A 0.3-ml portion of an appropriate dilution of the above calcium chloride-containing suspension was added to YT soft agar medium containing X-gal and IPTG (prepared by adding 10 µl of 100 mM IPTG, 50 µl of 2% X-gal and 0.2 ml of JM103 suspension at the logarithmic stage to 3 ml of a solution containing 0.6% agar, 0.8% tryptone, 0.5% yeast extract and 0.5% sodium chloride) while maintaining the medium at 45°C. The resultant mixture was spread onto YT agar medium (1.5% agar, 0.8% tryptone, 0.5% yeast extract, 0.5% sodium chloride). Incubation was conducted at 37°C for 16 hours. Six clones were selected from among the resultant white plaques, and each was inoculated into 2 x YT liquid medium (1.6% tryptone, 1% yeast extract, 1.0% sodium chloride), and incubation was conducted at 37°C for 8 hours. A one-milliliter portion of the culture broth was centrifuged at 12,000 rpm for 10 minutes, and the supernatant was recovered as a phage-containing liquid. To 25 µl of said phage-containing liquid, there was added 5 µl of a solution comprising 1% SDS, 40% glycerol and 0.2% bromophenol blue. After 10 minutes of warming at 65°C, the presence of the desired DNA (M13mp9-GEF146; Fig. 4) was confirmed on agarose gel, and the phage DNA (single-stranded DNA; ssDNA) was isolated and purified in the following way.

To 800 µl of the above phage-containing liquid, there was added 200 µl of 20% polyethylene glycol (PEG 6000) containing 2.5 M sodium chloride. After 15 minutes of standing at room temperature, the mixture was centrifuged at 12,000 rpm for 5 minutes for phage precipitation. The phage precipitate was suspended in 100 µl of TES buffer (10 mM Tris-hydrochloric acid buffer, pH 7.5, containing 1 mM EDTA and 150 mM sodium chloride). Following addition thereto of 50 µl of water-saturated phenol, the mixture was shaken vigorously for 5 minutes and then centrifuged at 10,000 rpm for 5 minutes. A 80-µl portion was taken from the aqueous phase, and 3 µl of 3 M sodium acetate and 200 µl of ethanol were added thereto to thereby cause DNA precipitation. The precipitate DNA recovered by centrifugation was washed once with ethanol and then dissolved in 50 µl of TES buffer.

b. Construction of GIF131 gene by in vitro mutation (cf. Fig. 5)

A DNA fragment having the sequence: 5'AAAACTGGTAAG TAAGTCGACTCTCAGATGCTGT3' was chemically synthesized by the phosphotriester method using the solid phase technique (cf. Japanese Kokai Tokkyo Koho No. 201995/1983). Then, 4 µg of this DNA was dissolved in 20 µl of kination buffer (70 mM Tris-hydrochloric acid buffer, pH 7.5, containing 10 mM magnesium chloride and 1 mM ATP) and, following addition of 4 units of polynucleotide kinase (Takara Shuzo), the phosphorylation of said chemically synthesized DNA fragment at the 5' end was effected by allowing the reaction to proceed at 37°C for 45 minutes.

To 2.5 µl of solution containing 0.5 µg of this 5' end-phosphorylated, chemically synthesized DNA fragment, there were added 2.5 µl of the phage DNA solution mentioned above (obtained in Example II-1-a) and 1.5 µl of Hin buffer (70 mM Tris-hydrochloric acid buffer, pH 7.5, containing 70 mM magnesium chloride and 500 mM sodium chloride), and the mixture was sealed in a glass capillary (100 µl micropipette was used), heated at 100°C for 3 minutes and then allowed to stand at 4°C for 2 hours. To this mixture, there were then added 2.5 µl of Hin buffer, 2.5 µl each of 5 mM dATP, dGTP, dCTP and dTTP, and 13.5 µl of distilled water, followed by further addition of 5 units of DNA polymerase I Klenow fragment (Boehringer Mannheim). The DNA synthesis reaction was then allowed to proceed at room temperature for 30 minutes. Thereafter, ATP was added to a concentration of 1 mM and, further, 5 units of T4 DNA ligase (Boehringer Mannheim) was added. After 18 hours of reaction at 14°C, 10 µl of the reaction mixture was used for transformation of Escherichia coli JM103 in the manner already mentioned hereinabove.

In the above-mentioned manner (described in Example II-1-a), 36 clones formed on YT soft agar medium were selected and each was inoculated into 2 x YT liquid medium. After 8 hours of incubation at 37°C, 1 ml of the culture was centrifuged at 12,000 rpm for 10 minutes and the supernatant was recovered as the phage-containing liquid. On the other hand, the cells obtained as a precipitate were subjected to extraction and isolation of RFDNA (double-stranded DNA) by the alkali extraction method (Brinboim, H. C. & Doly, J., Nucl. Acids Res., 7: 1513-1523, 1979). Clones were selected such that double digestion of said RFDNA with EcoRI and SalI gave an about 400 bp DNA fragment. Furthermore, clones were selected such that digestion of DNA from the above clones with BglII failed to cause cleavage of the DNA region corresponding to the GIF gene (cf. Fig. 6). From such clones (2 clones out of the 36 clones), the desired ones containing a GIF131-encoding DNA were obtained and named M13mp9-GIF131-26 and -34, respectively.

Each clone was purified in the following manner. For transformation, JM103 was infected with the phage-containing liquid previously obtained and corresponding to M13mp9-GIF131-26 or -34 and thereafter, in the same manner as mentioned above, RFDNA (double-stranded DNA) was isolated and analyzed by double digestion with EcoRI and SalI and digestion with BglII. This led to purification of the clone. Then, 0.8 ml of a culture of JM103 infected with said clone and 8 ml of a culture of uninfected JM103 were added to 800 ml of 2 x YT liquid medium, followed by incubation at 37°C for 5 hours. By centrifugation, a phage-containing liquid was obtained from the supernatant and an infected bacterial strain from the precipitate. RFDNA was obtained from the infected strain cells in the conventional manner by centrifugation on an ethidium bromide-containing cesium chloride density gradient. On the other hand, phage DNA was obtained from the phage-containing supernatant by the above-mentioned method, and the base sequence was determined by the dideoxy chain termination method (cf. Methods in Enzymology, vol. 65: 560-580, 1980, Academic Press, New York). As a result, it was confirmed that the clones examined (two clones) carried the base sequence as expected, namely the base sequence comprising the termination codon (TAA) and the SalI cleavage site directly following the GIF131-encoding DNA.

2. Construction of GIF131 gene expression vector

(cf. Fig. 6) and transformation

In 50 µl of TA buffer, there was dissolved 2.5 µg of pIN5GIF54 DNA, followed by addition of 10 units each of EcoRI and SalI. The mixture was warmed at 37°C for 1 hour to cause complete cleavage at the EcoRI and SalI sites. By using 1% agarose gel, a larger DNA fragment was isolated electrophoretically.

Separately, 5 µg of the previously-obtained M13mp9-GIF131-34 RFDNA was dissolved in 50 µl of TA buffer. Following addition of 20 units each of EcoRI and SalI, the solution was warmed at 37°C for 1 hour. Thereafter, an about 400 bp DNA fragment (GIF131 DNA) was isolated by electrophoresis using 1.5% agarose gel. This DNA fragment and the earlier-obtained DNA fragment were dissolved in 20 µl of ligation solution, followed by addition of 1 unit of T4 DNA ligase. After 16 hours of reaction at 14°C, 10 µl of the reaction mixture was mixed with a calcium chloride-treated suspension of Escheichia coli W3110 (0.3 ml of 50 mM calcium chloride) to thereby cause transformation of Escherichia coli W3110. The above liquid mixture, after appropriate dilution, was spread onto nutrient agar medium (Difco) containing 40 µg/ml of ampicillin and incubation was conducted at 37°C overnight. From among the resultant colonies, 12 colonies were chosen in an adequate manner and cultured in L broth containing 40 µg/ml of ampicillin at 37°C for 18 hours and then the plasmid DNA was isolated and purified by the alkali extraction method mentioned above. It was confirmed that the plasmid DNA obtained gave an about 400 bp DNA fragment upon double digestion with EcoRI and SalI but was not cleaved with BglII. The 12 transformants chosen all carried the desired plasmid. This plasmid was named pIN5GIF54-131 and the strain Escherichia coli W3110 transformed with this plasmid was named W3110/pIN5GIF54-131.

When the strain WA802 is used as the host Escherichia coli strain in place of W3110, a transformant, WA802/pIN5GIF54-131, in which the GIF131 gene is expressed, can be obtained in the same manner as above. It is also possible to obtain a GIF131-producing transformant called WA802/pYtacGIF-131 or W3110/pYtacGIF-131 by constructing a GIF131 gene expression vector, pYtacGIF-131, using pYtacGIF (Japanese Patent Application No. 162337/1983) in lieu of the pIN5GIF54 plasmid in Fig. 6 and following the same procedure as above and then using the plasmid for transformation of the strain WA802 or W3110, respectively.

All the restriction enzymes used in this example were purchased from Takara Shuzo.

3. Production of GIF131 by W3110/pIN5GIF54-131

The Escherichia coli transformant W3110/pIN5GIF54-131 was inoculated into 2 liters of M9 medium for E. coli containing casamino acids (3%), followed by incubation with aeration at 30°C for 24 hours. Thereafter, about 60 g of wet cells were obtained by centrifugation. The cells were washed with 50 mM phosphate buffer (pH 6.0), then suspended in the same buffer and disrupted under cooling (10°C or below) using a Manton Gaulin homogenizer (already mentioned). The homogenate was separated into a precipitate and a supernatant by centrifugation (7,000 rpm, 20 minutes). The supernatant was assayed for antiviral activity (IFN activity). (The method of IFN activity measurement is mentioned later.) The activity was $5 \times 10^4$ U/ml.

The above supernatant and various standard proteins (Pharmacia's standard proteins kit for electrophoresis, Lot No. 2007) were subjected to 13% polyacrylamide gel electrophoresis. A dsitinct, strong band of protein was observed at a location corresponding to a molecular weight of about 16,000. The absence of this band for the control (Escherichia coli carrying no pIN5GIF54-131 plasmid) and the coincidence in location (distance of travel) between said band and the band observed in Example IV-1-a (Fig. 9) were confirmative of the fact that W3110/pIN5GIF54-131 was actually a GIF131 polypeptide-producing strain. It is obvious that the purification of GIF131 from the supernatant obtained in this example can be carried out by the method described in Example I-2.

III. Assay of purified polypeptide for purity

The purity of the polypeptide fraction having IFN activity as purified by CM cellulose C-52 chromatography was checked in the following manner:

1. SDS electrophoresis

Said purified polypeptide fraction was subjected to SDS electrophoresis by the conventional method at an acrylamide concentration of 13%. A single band was found at the position corresponding to a molecular weight of about 16,000 (16 K). It was thus revealed that said fraction had been highly purified.

Furthermore, silver staining [Tanpakushitsu Koso Kiso Jikkenho (Fundamental Methods of Experimentation with Proteins and Enzymes), edited by Horio-Yamashita, p. 439, 1982, Nankodo] confirmed that said fraction contained almost no impurities.

2. High performance liquid chromatography

Said fraction was subjected to high performance liquid chromatography (using a Waters' chromatograph) using a Waters micro Bondapak C18 column. The elution of the polypeptide in question was effected by using acetonitrile solution adjusted to pH 2.0 with trifluoroacetic acid and increasing the acetonitrile concentration from 23% to 69% (Fig. 7). A single peak was observed at an acetonitrle concentration of about 53%. The polypeptide was detected based on the absorption at 214 nm (A214).

IV Physico-chemical properties of purified polypeptide

The polypeptide according to the invention was examined for the following physico-chemical properties:

1. Molecular weight

The molecular weight of the purified polypeptide was determined by polyacrylamide SDS electrophoresis (SDS PAGE) and by molecular sieve high-perfromance liquid chromatography.

a. <u>SDS PAGE</u>

For molecular weight estimation, various standard proteins (Pharmacia's standard proteins kit for electrophoresis, Lot No. 2007), the GIF146 polypeptide, and the purified polypeptide according to the invention were subjected to 13% acrylamide SDS electrophoresis (cf. Fig. 8). Fig. 8-a shows the results of electrophoresis, the pattern A being for the GIF146 polypeptide, B, D and F for standard proteins (94 K: phosphorylase B; 67 K: bovine serum albumin; 43 K: eggwhite albumin; 30 K: carbonic anhydrase; 20 K: soybean trypsin inhibitor; 14.4 K: α-lactoalbumin), C and E for the purified polypeptide of the invention, and G for a mixture of the GIF146 polypeptide and the polypeptide of the invention. Fig. 8-b is a graphic representation of the distance of electrophoretic travel of each protein or polypeptide. Based on this graphic representation, the molecular weight of the polypeptide of the invention was estimated at about 16,000 (16 K).

b. <u>High performance liquid chromatography (HPLC)</u>

Based on the graphic representation (Fig. 9) of the elution patterns for standard proteins (Oriental Yeast's kit) and the polypeptide of the invention in high performance liquid chromatography using a GPC column G-3000SW (Toyo Soda) (solvent: 0.1 M potassium phosphate containing 0.3 M NaCl; rate of flow: 0.7 ml/minute), the apparent molecular weight of the polypeptide of the invention was estimated at about 17,000 (17 K).

2. Isoelectric point

The isoelectric point (PI value) of the polypeptide according to the invention was measured by the isoelectrofocusing method and plate method (cf. Vesterberg, O. & Svensson, H., Acta Chem. Scand., 20: 820, 1966).

The isoelectrofocusing was conducted using Ampholine pH 9-11 and a model 110 column (both from LKB, Sweden) and following the method of Matsuo et al. (Protein, Nucleic Acid and Enzyme, Vol. 12, No. 9, p. 737, 1967, Kyoritsu Shuppan) (cf. Fig. 10). As shown in Fig. 10, the IFN activity of said polypeptide was noted in fractions having a pH of about 9.2 to 9.4, and the average isoelectric point (PI value) was estimated at about 9.3.

The plate method was performed on an Ampholine acrylamide gel at pH 7.8 to 10.0 using an LKB Multipho electrophoresing apparatus. The main isoelectric point (PI value) found of the polypeptide of the invention was about 9.8. Small amounts of bands were also found on the locations corresponding to pI values of 9.3 and 10.0. This is supposedly due to polymerization of the present polypeptide.

3. Amino acid composition

The purified polypeptide according to the invention (39 µg) was desalted and then dissolved in hydrochloric acid for amino acid analysis. The thus-prepared 6 N HCl solution was sealed in a tube under vacuum and subjected to hydrolysis in an electric oven at 110°C for 24, 48 or 72 hours. Then, the hydrochloric acid was removed under reduced pressure and the residue was subjected to amino acid analysis using a Hitachi model 835-50 amino acid analyzer. The Leu (leucine) content was taken as the basis and the amino acid composition was calculated on the assumption that the molecular weight was 16,000, the results of which are shown in Table 1. The theoretical values are the values for the polypeptide described in Claim 2 and composed of 131 amino acid residues (X being a hydrogen atom).

Table 1

| Amino acid | Amino acid composition | |
|---|---|---|
| | Found | Theoretical** |
| $CySO_3H$[a] | 1.85 | 2 |
| Asp | 19.70 | 20 |
| Thr[b] | 5.00 | 5 |
| Ser[b] | 8.90 | 9 |
| Glx | 15.56 | 16 |
| Pro | 2.27 | 2 |
| Gly | 4.10 | 4 |
| Ala | 7.09 | 7 |
| Cys | - | - |

| | | |
|---|---|---|
| Val[c] | 7.99 | 8 |
| Met[b] | 2.89 | 3 |
| Ile[c] | 6.87 | 7 |
| Leu | 9.00* | 9 |
| Tyr | 4.85 | 5 |
| Phe | 8.85 | 9 |
| Lys | 18.47 | 19 |
| His | 2.06 | 2 |
| Arg | 3.40 | 3 |
| Trp[d] | - | 1 |

* Leu taken as the basis.

** Calculated from the values after 24 hours and 72 hours of hydrolysis.

a Analysis made after oxidation with formic acid.

b Calculated by extrapolation to time 0 (zero).

c Value after 72 hours of hydrolysis.

d Cannot be determined under the conditions of this experiment.

4. Amino terminal analysis

Said purified polypeptide was dissolved in hydrochloric acid-acidified methanol to thereby convert all formylated Met (methionine) residues to the free Met form. Thereafter, the amino terminal was degraded with 5% hydrochloric acid-containing methanol, and the amino acids liberated were analyzed using the above-mentioned amino acid analyzer. It was estimated that Met (inclusive of formylated Met) accounted for about 30% of the amino-

terminal amino acids.

5. Analysis of amino acid sequence (primary structure) and carboxyl terminal

Said purified polypeptide (1 mg) was dissolved in 0.1 ml of 70% formic acid containing 1.8 mg of cyanogen bromide (BrCN), and Met contained in said polypeptide was selectively cleaved by 24 hours of reaction at room temperature. The formic acid and cyanogen bromide were then removed under reduced pressure, and the residue was dissolved in 10% aqueous acetonitrile solution containing trifluoroacetic acid in an amount to make pH 2.0. The solution was subjected to high performance liquid chromatography (Waters) using a μ-Bondapak C18 column (Waters), fractionation being effected by increasing the acetonitrile concentration to 75%. With three eluate fractions [I: BrCN (49-80); II: BrCN (81-120); and III: BrCN (121-131)] and the polypeptide before BrCN decomposition, the amino acid sequence was analyzed by the Edman method using an amino acid sequence analyzer (Upright Biosystem model 470A).

The results are shown in Fig. 11. In the figure, the arrows indicate where the amino acid sequence has been established in the present experiment. Where there are no arrows, the amino acid sequence described in Claim 2 is given.

Analysis of the amino acid formed by decomposition of said purified polypeptide by the carboxypeptidase method revealed that the carboxyl-terminal amino acid is Lys (lysine).

The results of the above experiments (in particular Examples III-3, III-4 and III-5) and the method of production of this substance strongly suggest that the polypeptide according to the invention is a polypeptide (GIF131 for short) composed of 131 or 132 amino acid residues as shown in Claim 2.

6. Elemental analysis

Said purified polypeptide (2 mg) was desalted by dialyzing against distilled water and then lyophilized. A 1.204-mg portion of the lyophilizate was subjected to elemental analysis for C, N and H using a Perkin-Elmer model 240B elemental analyzer. The results obtained are shown in Table 2.

Table 2

| | Found | Calculated* |
|---|---|---|
| C | 48.98% | 53.74% |
| H | 6.67% | 7.06% |
| N | 15.02% | 16.59% |

* Calculated on the basis of the polypeptide composed of 131 amino acid residues as described in Claim 2. Thus, calculated based for $C_{684}H_{1671}O_{206}N_{181}S_5$.

In Table 2, the values found are slightly lower than the calculated values. However, if the ratio among C, H and N is calculated, then the measured value ratio: C:H:N = 1:0.136:0.307 is in fairly good agreement with the theoretical ratio: C:H:N = 1:0.131:0.309.

Generally, lyophilizates of high-molecular-weight peptides or proteins may possible contain water and/or buffer components used in the course of purification. The difference between the measured value and the theoretical value as found in this elemental analysis is presumably due to such fact.

7. Ultraviolet absorption spectrum

Said purified polypeptide was dissolved in 15 mM ammonium formate to a concentration of 0.23 mg/ml and the solution was measured for its absorption spectrum in the range of from 340 nm to 190 nm (using a Shimadzu model UV 240 spectrophotometer). The results obtained are shown in Fig. 12.

8. Circular dichroism

Said purified polypeptide was dissolved in 0.15 M ammonium formate in a concentration of 0.1 mg/ml, and the solution was measured for grade 2 circular dichroism using a 0.1-cm cell (using a Nippon Bunkou Kogyo model J-20C). The results obtained are shown in Fig. 13.

9. Nuclear magnetic resonance (NMR) spectrum

Said purified polypeptide (1 mg) was desalted and lyophilized, and dissolved in 0.7 ml of $D_2O$. The solutic was measured for NMR spectrum at a frequency of 360 MH (megahertz) using a Nicolet model NT360 NMR spectrometer The results obtained are shown in Fig. 14.

10. Infrared absorption spectrum

Said purified polypeptide (0.3 mg) was desalted and lyophilized, and measured for its infrared absorption spectrum by the KBr disc method. A Nicolet model 5-DX FT-IR apparatus was used and the measurement was conducted in the wavelength range of 4600 nm to 400 nm. The results obtained are shown in Fig. 15.

V Biological activity

1. Measurement of antiviral activity

As already mentioned, the polypeptide according to the invention has potent interferon activity. The interferon activity of said polypeptide was measured in terms of antiviral activity, as mentioned hereinbelow.

The antiviral activity measurement was performed by the CPE50 method (cytopathic effect method; The Clinical Potential of Interferon, p. 299-309, 1980, Tokyo University Press) using FL amnion and Sindbis virus.

As the standard antiviral substances for the assay, there were used a crude interferon solution (mostly gamma-type interferon) formed upon induction by adding SES (Staphylococcus endotoxin B) to human leukocytes, and purified alpha-type interferon.

The antiviral activity of the polypeptide of the present invention is not lost upon neutralization reaction with human α-type and β-type interferon antibodies but is neutralized by human γ-type interferon antibody.

Furthermore, with said polypeptide, a precipitate line is observable only with anti-human γ-type interferon sera in immunoelectrophoresis using a thin-layer agarose gel [Men-ekikagku Doteiho (Identification Methods in Immunochemistry), edited by Sasaki and Muraji, Tokyo University Press, 1973, p. 25-28]. Based on these facts, the polypeptide (GIF131) according to the invention can be said to be a human gamma-type interferon-like substance.

2. Stability of biological activity

It is generally said that γ-type interferons are readily inactivated upon treatment at a low pH or with SDS. Therefore, the purified polypeptide according to the invention was examined for acid resistance, alkali resistance, heat resistance and SDS sensitivity of its biological activity, among others.

a. Acid and alkali resistance

The purified polypeptide according to the invention was dissolved in buffers varying in pH in a concentration of 10 μg/ml. The solutions were allowed to stand at 4°C for 0-14 days, then adjusted to a neutral pH by dilution with 0.1 M phosphate buffer (pH 7.0), and assayed for residual antiviral activity. The results obtained are shown in Table 3.

Table 3

| Buffer | pH | Residual antiviral activity (%)* Day 0 | Day 3 | Day 7 | Day 14 |
|---|---|---|---|---|---|
| SAB[a)] | 5.0 | 33 | 16 | 16 | 8 |
| | 5.5 | 100 | 32 | 32 | 16 |
| SPB[b)] | 6.0 | 100 | 63 | 32 | 32 |
| | 6.5 | 100 | 63 | 63 | 63 |
| | 7.0 | 100 | 63 | 63 | 63 |
| THB[c)] | 7.5 | 100 | 63 | 63 | 63 |
| | 8.0 | 58 | 32 | 32 | 32 |
| | 8.5 | 58 | 32 | 32 | 16 |
| | 9.0 | 58 | 32 | 32 | 16 |
| Control** | 7.2 | 100 | 63 | 63 | 63 |

* Calculated taking the residual activity in the control as 100.

** The buffer in the control was phosphate buffer containing 0.15 M NaCl.

a) Sodium acetate buffer

b) Sodium phosphate buffer

c) Tris-hydrochloride buffer

Table 4

| pH | Residual antiviral activity (%) Hour 0 | Hour 2 | Hour 12 |
|---|---|---|---|
| 2.0 | 100 | 30 | 20 |
| 7.2 (Control) | 100 | 100 | 100 |

* Calculated taking the residual activity in the control (pH 7.2) as 100.

Then, for checking the biological stability of said purified polypeptide at low pH (pH 2.0), 10 μg of said polypeptide was dissolved in distilled water, followed by adjustment to pH 2.0 with 1 N hydrochloric acid, to make 1 ml of solution. The solution was allowed to stand at 4°C for 0-12 hours, then neutralized with 1 N NaOH, and assayed for residual antiviral activity. As shown in Table 4, the results obtained indicated that about 20% of the antiviral activity remained even after 12 hours of treatment, suggesting that said polypeptide be more acid-resistant than natural IFN-γ (GIF146).

b. SDS (sodium dodecyl sulfate) sensitivity

Said purified polypeptide was dissolved in 0.1% aqueous SDS solution in a concentration of 10 μg/ml. The solution was allowed to stand at 4°C for 0-12 hours, then diluted with an adequate amount of distilled water and assayed for residual antiviral activity. The results obtained are shown in Table 5. The residual antiviral activity was about 20% after 2 hours and about 10% even after 12 hours. It was thus suggested that said polypeptide be more resistant to SDS than natural IFN-γ (GIF146).

Table 5

| Treatment | Residual antiviral activity (%)* | | |
|---|---|---|---|
| | Hour 0 | Hour 2 | Hour 12 |
| 0.1% | 100 | 20 | 10 |
| Distilled water (control) | 100 | 100 | 100 |

* Calculated based on the residual activity in the control (distilled water) which activity was taken as 100.

Japanese Patent Applications Nos. 86180/1982, 132524/1983 and 162337/1983 referred to herein correspond to European Patent Applications Nos. 83302915.0, 84304901.6 and 84305996.5 respectively.

Strains WA 802 and W3110 are available from E. Coli Genetic Stock Control (USA).

Claims:

1. A novel basic polypeptide consisting of 131 or 132 amino acid residues and having an immune interferon-like activity, said polypeptide being characterized by: an apparent molecular weight of about 16,000 as measured by SDS electrophoresis;

an apparent molecular weight of about 17,000 as measured by the high performance liquid chromatographic molecular sieve method;

an isoelectric point (PI value) in the range of 9.2-10.0;

a cystein or methionine residue as the amino terminal;

a lysine residue as the carboxyl terminal; and

the amino acid composition: Asx 20, Thr 5, Ser 9, Glx 16, Pro 2, Gly 4, Ala 7, Cys 2, Val 8, Met 3 or 4, Ile 7, Leu 9, Tyr 5, Phe 9, Lys 19, His 2, Arg 3 and Trp 1 (Asx including Asp and Asn and Glx including Glu and Gln).

2. A basic polypeptide of Claim 1, which has the following amino acid sequence:

```
      1
X    Cys Tyr Cys Gln Asp Pro Tyr Val Lys
     10
     Glu Ala Glu Asn Leu Lys Lys Tyr Phe
         20
     Asn Ala Gly His Ser Asp Val Ala Asp
             30
     Asn Gly Thr Leu Phe Leu Gly Ile Leu
                 40
     Lys Asn Trp Lys Glu Glu Ser Asp Arg
```

```
                50
Lys Ile Met Gln Ser Gln Ile Val Ser
                    60
Phe Tyr Phe Lys Leu Phe Lys Asn Phe
                        70
Lys Asp Asp Gln Ser Ile Gln Lys Ser
                            80
Val Glu Thr Ile Lys Glu Asp Met Asn
                                90
Val Lys Phe Phe Asn Ser Asn Lys Lys

Lys Arg Asp Asp Phe Glu Lys Leu Thr
100
Asn Tyr Ser Val Thr Asp Leu Asn Val
    110
Gln Arg Lys Ala Ile His Glu Leu Ile
        120
Gln Val Met Ala Glu Leu Ser Pro Ala
            130
Ala Lys Thr Gly Lys
```

wherein X is a hydrogen atom or a methionyl group.

3. A polypeptide of Claim 1 or 2 obtained from the culture of a microorganism transformed with a plasmid containing a chemically synthesized DNA sequence coding for the following amino acid sequence:

```
    1
Met Cys Tyr Cys Gln Asp Pro Tyr Val Lys
    10
    Glu Ala Glu Asn Leu Lys Lys Tyr Phe
        20
    Asn Ala Gly His Ser Asp Val Ala Asp
            30
    Asn Gly Thr Leu Phe Leu Gly Ile Leu
                40
    Lys Asn Trp Lys Glu Glu Ser Asp Arg
                    50
    Lys Ile Met Gln Ser Gln Ile Val Ser
                        60
    Phe Tyr Phe Lys Leu Phe Lys Asn Phe
                            70
    Lys Asp Asp Gln Ser Ile Gln Lys Ser
                                80
    Val Glu Thr Ile Lys Glu Asp Met Asn
                                    90
    Val Lys Phe Phe Asn Ser Asn Lys Lys

    Lys Arg Asp Asp Phe Glu Lys Leu Thr
```

```
100
Asn Tyr Ser Val Thr Asp Leu Asn Val
    110
Gln Arg Lys Ala Ile His Glu Leu Ile
        120
Gln Val Met Ala Glu Leu Ser Pro Ala
            130
Ala Lys Thr Gly Lys
```

4. A polypeptide of Claim 3, wherein the plasmid is pIN5GIF54-131 or pYtacGIF131.

5. A polypeptide of Claim 1 or 2 obtained from the culture of a microorganism transformed with a plasmid containing a chemically synthesized DNA sequence coding for the following amino acid sequence:

```
     1
Met Cys Tyr Cys Gln Asp Pro Tyr Val Lys
    10
    Glu Ala Glu Asn Leu Lys Lyr Tyr Phe
        20
    Asn Ala Gly His Ser Asp Val Ala Asp
            30
    Asn Gly Thr Leu Phe Leu Gly Ile Leu
                40
    Lys Asn Trp Lys Glu Glu Ser Asp Arg
                    50
    Lys Ile Met Gln Ser Gln Ile Val Ser
                        60
    Phe Tyr Phe Lys Leu Phe Lys Asn Phe
                            70
    Lys Asp Asp Gln Ser Ile Gln Lys Ser
                                80
    Val Glu Thr Ile Lys Glu Asp Met Asn
                                    90
    Val Lys Phe Phe Asn Ser Asn Lys Lys

    Lys Arg Asp Asp Phe Glu Lys Leu Thr
    100
    Asn Tyr Ser Val Thr Asp Leu Asn Val
        110
    Gln Arg Lys Ala Ile His Glu Leu Ile
            120
    Gln Val Met Ala Glu Leu Ser Pro Ala
                130
    Ala Lys Thr Gly Lys Arg Lys Arg Ser
                    140
    Gln Met Leu Phe Arg Gly Arg Arg Ala
        146
    Ser Gln
```

6. A polypeptide of Claim 5, wherein the plasmid is pIN5GIF54 or pYtacGIF.

7. A polypeptide of Claim 3 or 5, wherein the transformant microorganism belongs to the species Escherichia coli.

8. A polypeptide of Claim 7, wherein the Eschericia coli transformant is W3110/pIN5GIF54 or WA802/pIN5GIF54.

9. A polypeptide of Claim 7, wherein the Escherichia coli transformant is WA802/pYtacGIF or W3110/pYtacGIF.

10. A polypeptide of Claim 7, wherein the Escherichia coli transformant is W3110/pIN5GIF54-131 or WA802/pIN5GIF54-131.

11. A polypeptide of Claim 7, wherein the Escherichia coli transformant is W3110/pYtacGIF131 or WA802/pYtacGIF131.

12. A method of producing the polypeptide of Claim 1 or 2, which comprises growing a microorganism transformed with a plasmid containing a chemically synthesized DNA sequence coding for the amino acid sequence:

```
    1
Met Cys Tyr Cys Gln Asp Pro Tyr Val Lys
    10
    Glu Ala Glu Asn Leu Lys Lys Tyr Phe
        20
    Asn Ala Gly His Ser Asp Val Ala Asp
            30
    Asn Gly Thr Leu Phe Leu Gly Ile Leu
```

```
                 40
Lys Asn Trp Lys Glu Glu Ser Asp Arg
                     50
Lys Ile Met Gln Ser Gln Ile Val Ser
                         60
Phe Tyr Phe Lys Leu Phe Lys Asn Phe
                             70
Lys Asp Asp Gln Ser Ile Gln Lys Ser
                                 80
Val Glu Thr Ile Lys Glu Asp Met Asn
                                     90
Val Lys Phe Phe Asn Ser Asn Lys Lys

Lys Arg Asp Asp Phe Glu Lys Leu Thr
100
Asn Tyr Ser Val Thr Asp Leu Asn Val
    110
Gln Arg Lys Ala Ile His Glu Leu Ile
        120
Gln Val Met Ala Glu Leu Ser Pro Ala
            130
Ala Lys Thr Gly Lys,
```

extracting said polypeptide from the culture of said microorganism, and purifying the same.

13. A method of Claim 12, wherein the plasmid is pIN5GIF54-131 or pYtacGIF131.

14. A method of Claim 12, wherein the chemically synthesized DNA has the following DNA sequence:

```
5'AATTC ATG TGC TAC TGC CAG GAC CCA TAC
   3' G TAC ACG ATG ACG GTC CTG GGT ATG

GTG AAG GAA GCT GAA AAC CTG AAG AAA TAC
CAC TTC CTT CGA CTT TTG GAC TTC TTT ATG

TTC AAC GCT GGT CAT TCT GAC GTT GCT GAC
AAG TTG CGA CCA GTA AGA CTG CAA CGA CTG

AAC GGT ACT CTG TTC CTG GGT ACT CTG AAA
TTG CCA TGA GAC AAG GAC CCA TAG GAC TTT

AAC TGG AAA GAA GAA TCT GAC CGT AAA ATC
TTG ACC TTT CTT CTT AGA CTG GCA TTT TAG
```

ATG CAG TCT CAG ATC GTT TCT TTC TAC TTC
TAC GTC AGA GTC TAG CAA AGA AAG ATG AAG

AAG CTG TTC AAA AAC TTC AAG GAC GAC CAG
TTC GAC AAG TTT TTG AAG TTC CTG CTG GTC

TCT ATC CAG AAA TCT GTT GAA ACT ATC AAG
AGA TAG GTC TTT AGA CAA CTT TGA TAG TTC

GAA GAC ATG AAC GTT AAG TTC TTC AAC TCT
CTT CTG TAC TTG CAA TTC AAG AAG TTG AGA

AAC AAG AAA AAG CGT GAC GAC TTC GAA AAG
TTG TTC TTT TTC GCA CTG CTG AAG CTT TTC

CTT ACT AAC TAC TCT GTT ACT GAC CTT AAT
GAA TGA TTG ATG AGA CAA TGA CTG GAA TTA

GTA CAG CGT AAA GCT ATC CAT GAA CTG ATC
CAT GTC GCA TTT CGA TAG GTA CTT GAC TAG

CAG GTT ATG GCT GAA CTG TCC CCG GCT GCT
GTC CAA TAC CGA CTT GAC AGG GGC CGA CGA

AAA ACT GGT AAG TAA C 3'
TTT TGA CCA TTC ATT CAGCT 5',

wherein the sequence in the square □ may alternatively be $\frac{TGT}{ACA}$.

15. A method of producing the polypeptide of Claim 1 or 2, which comprises growing a microorganism transformed with a plasmid containing a chemically synthesized DNA sequence coding for the amino acid sequence:

1
Met Cys Tyr Cys Gln Asp Pro Tyr Val Lys
10
Glu Ala Glu Asn Leu Lys Lys Tyr Phe
20
Asn Ala Gly His Ser Asp Val Ala Asp
30
Asn Gly Thr Leu Phe Leu Gly Ile Leu
40
Lys Asn Trp Lys Glu Glu Ser Asp Arg
50
Lys Ile Met Gln Ser Gln Ile Val Ser
60
Phe Tyr Phe Lys Leu Phe Lys Asn Phe
70
Lys Asp Asp Gln Ser Ile Gln Lys Ser
80
Val Glu Thr Ile Lys Glu Asp Met Asn
90
Val Lys Phe Phe Asn Ser Asn Lys Lys

Lys Arg Asp Asp Phe Glu Lys Leu Thr
100
Asn Tyr Ser Val Thr Asp Leu Asn Val Gln
110
Arg Lys Ala Ile His Glu Leu Ile Gln Val
120
Met Ala Glu Leu Ser Pro Ala Ala Lys Thr
130
Gly Lys Arg Lys Arg Ser Gln Met Leu Phe
140 146
Arg Gly Arg Arg Ala Ser Gln,

extracting said polypeptide from the culture of said microorganism, and purifying the same.

16. A method of Claim 15, wherein the plasmid is pIN5GIF54 or pYtacGIF.

17. A method of Claim 15, wherein the chemically synthesized DNA has the following DNA sequence:

```
5'AATTC ATG |TGC| TAC TGC CAG GAC CCA TAC
   3' G TAC |ACG| ATG ACG GTC CTG GGT ATG

GTG AAG GAA GCT GAA AAC CTG AAG AAA TAC
CAC TTC CTT CGA CTT TTG GAC TTC TTT ATG

TTC AAC GCT GGT CAT TCT GAC GTT GCT GAC
AAG TTG CGA CCA GTA AGA CTG CAA CGA CTG

AAC GGT ACT CTG TTC CTG GGT ACT CTG AAA
TTG CCA TGA GAC AAG GAC CCA TAG GAC TTT

AAC TGG AAA GAA GAA TCT GAC CGT AAA ATC
TTG ACC TTT CTT CTT AGA CTG GCA TTT TAG

ATG CAG TCT CAG ATC GTT TCT TTC TAC TTC
TAC GTC AGA GTC TAG CAA AGA AAG ATG AAG

AAG CTG TTC AAA AAC TTC AAG GAC GAC CAG
TTC GAC AAG TTT TTG AAG TTC CTG CTG GTC

TCT ATC CAG AAA TCT GTT GAA ACT ATC AAG
AGA TAG GTC TTT AGA CAA CTT TGA TAG TTC

GAA GAC ATG AAC GTT AAG TTC TTC AAC TCT
CTT CTG TAC TTG CAA TTC AAG AAG TTG AGA

AAC AAG AAA AAG CGT GAC GAC TTC GAA AAG
TTG TTC TTT TTC GCA CTG CTG AAG CTT TTC

CTT ACT AAC TAC TCT GTT ACT GAC CTT AAT
GAA TGA TTG ATG AGA CAA TGA CTG GAA TTA

GTA CAG CGT AAA GCT ATC CAT GAA CTG ATC
CAT GTC GCA TTT CGA TAG GTA CTT GAC TAG

CAG GTT ATG GCT GAA CTG TCC CCG GCT GCT
GTC CAA TAC CGA CTT GAC AGG GGC CGA CGA

AAA ACT GGT AAG CGT AAA AGA TCT CAG ATG
TTT TGA CCA TTC GCA TTT TCT AGA GTC TAC
```

CTG TTC CGT GGT CGT CGT GCT TCT CAG TAA
GAC AAG GCA CCA GCA GCA CGA AGA GTC ATT

G 3'
CAGCT 5', wherein the sequence in the square □ may alternatively be TGT/ACA.

18. A method of any one of Claims 12, 14, 15 and 16, wherein the transformant microorganism belongs to the species <u>Escherichia coli</u>.

19. A method of Claim 18, wherein the <u>Escherichia coli</u> transformant is W3110/pIN5GIF54 or WA802/pIN5GIF54.

20. A method of Claim 18, wherein the <u>Escherichia coli</u> transformant is W3110/pYtacGIF or WA802/pYtacGIF.

21. A method of Claim 18, wherein the <u>Escherichia coli</u> transformant is W3100/pIN5GIF54-131 or WA802/pIN5GIF54-131.

22 A method of Claim 18, wherein the <u>Escherichia coli</u> transformant is W3100/pYtacGIF131 or WA802/pYtacGIF131.

23. A polypeptide as claimed in Claim 1 or Claim 2, for use as an antitumor, anticancer, antiviral or immunomodulating agent in the treatment of the human or animal body.

24. An antitumor, anticancer, antiviral or immunomodulating composition comprising a polypeptide as claimed in Claim 1 or Claim 2 as an antitumor, anticancer, antiviral or immunomodulating agent.

0147175

1/15

Fig. 1

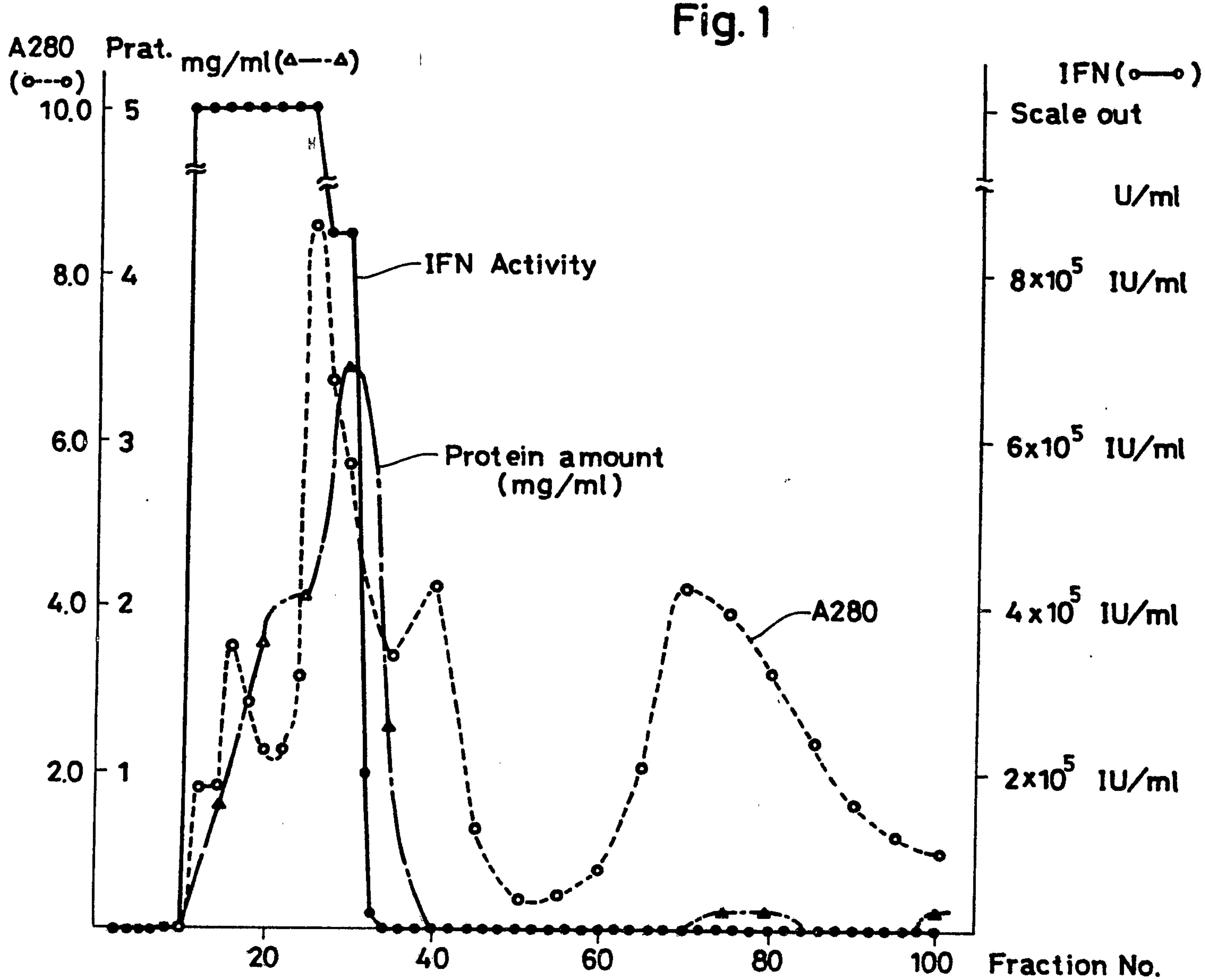

0147175

2/15

Fig. 2

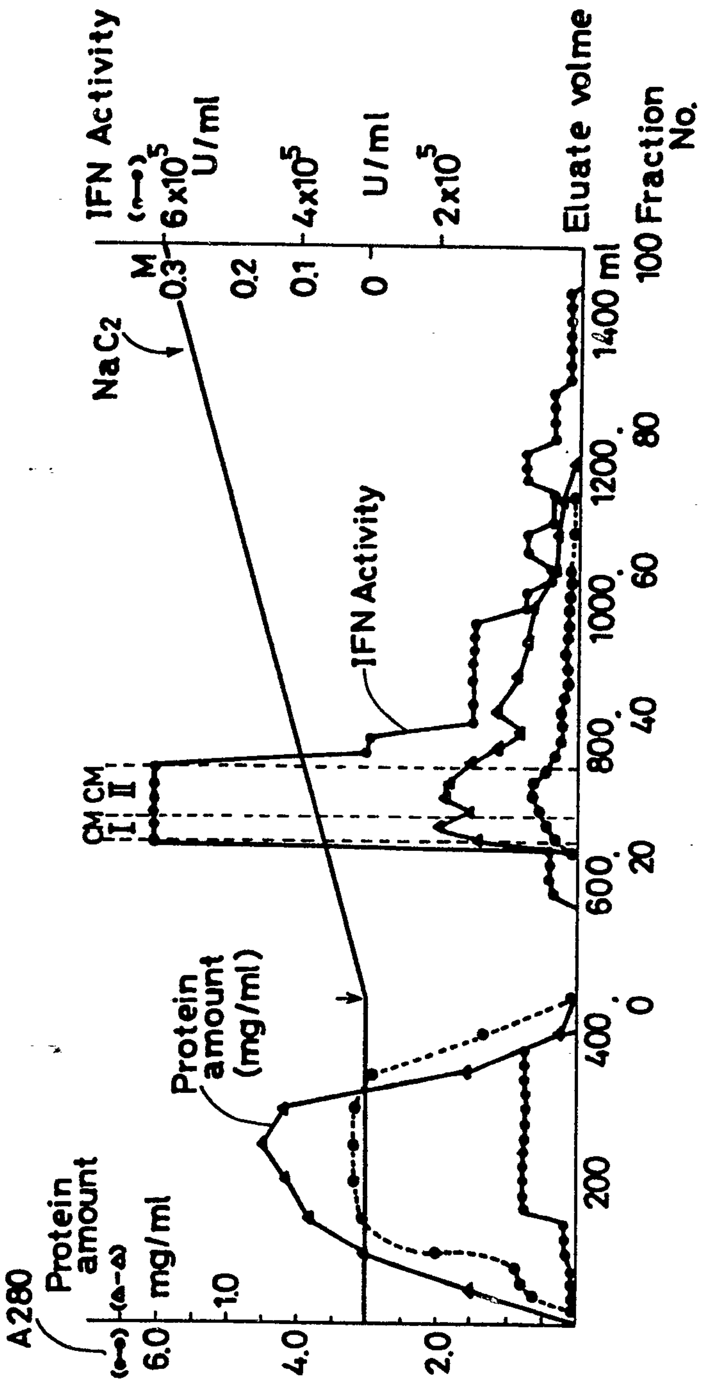

Fig. 3

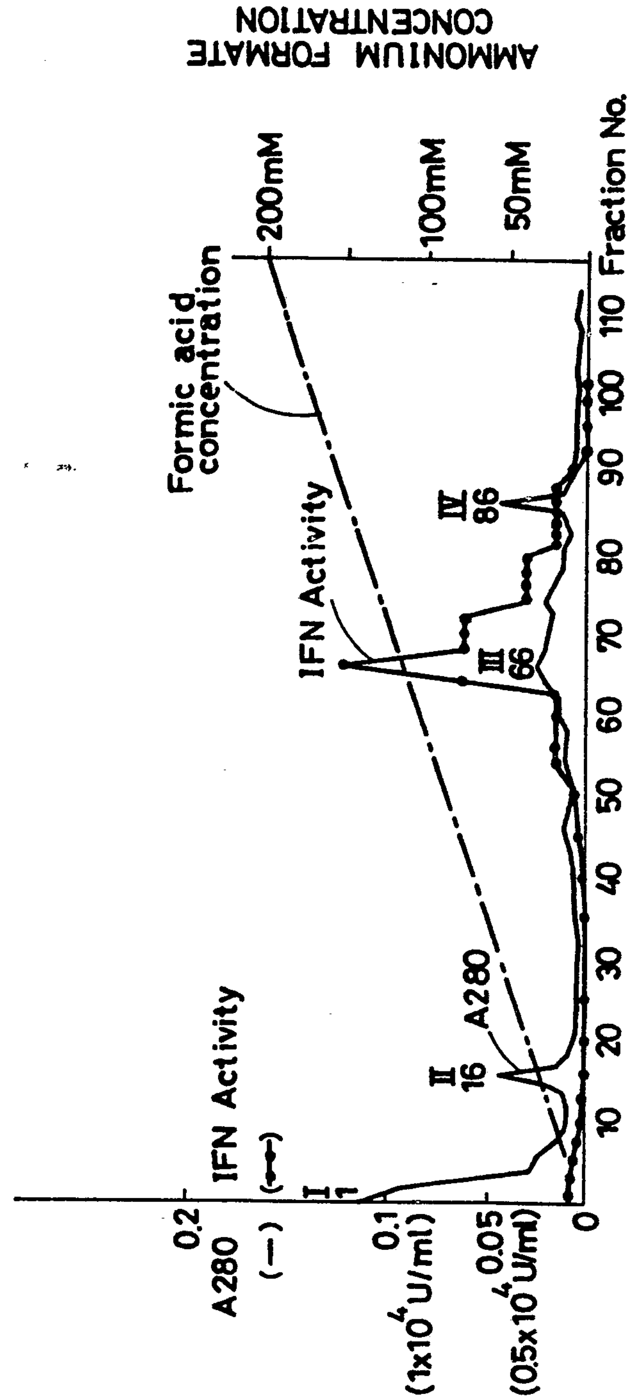
AMMONIUM FORMATE CONCENTRATION
200mM
100mM
50mM
Fraction No.
Formic acid concentration
IFN Activity
A280
IFN Activity (—•—)
A280 (—)
0.2
0.1 (1x10⁴ U/ml)
0.05 (0.5x10⁴ U/ml)
0
I 1
II 16
III 66
IV 86
10
20
30
40
50
60
70
80
90
100
110

Fig. 4

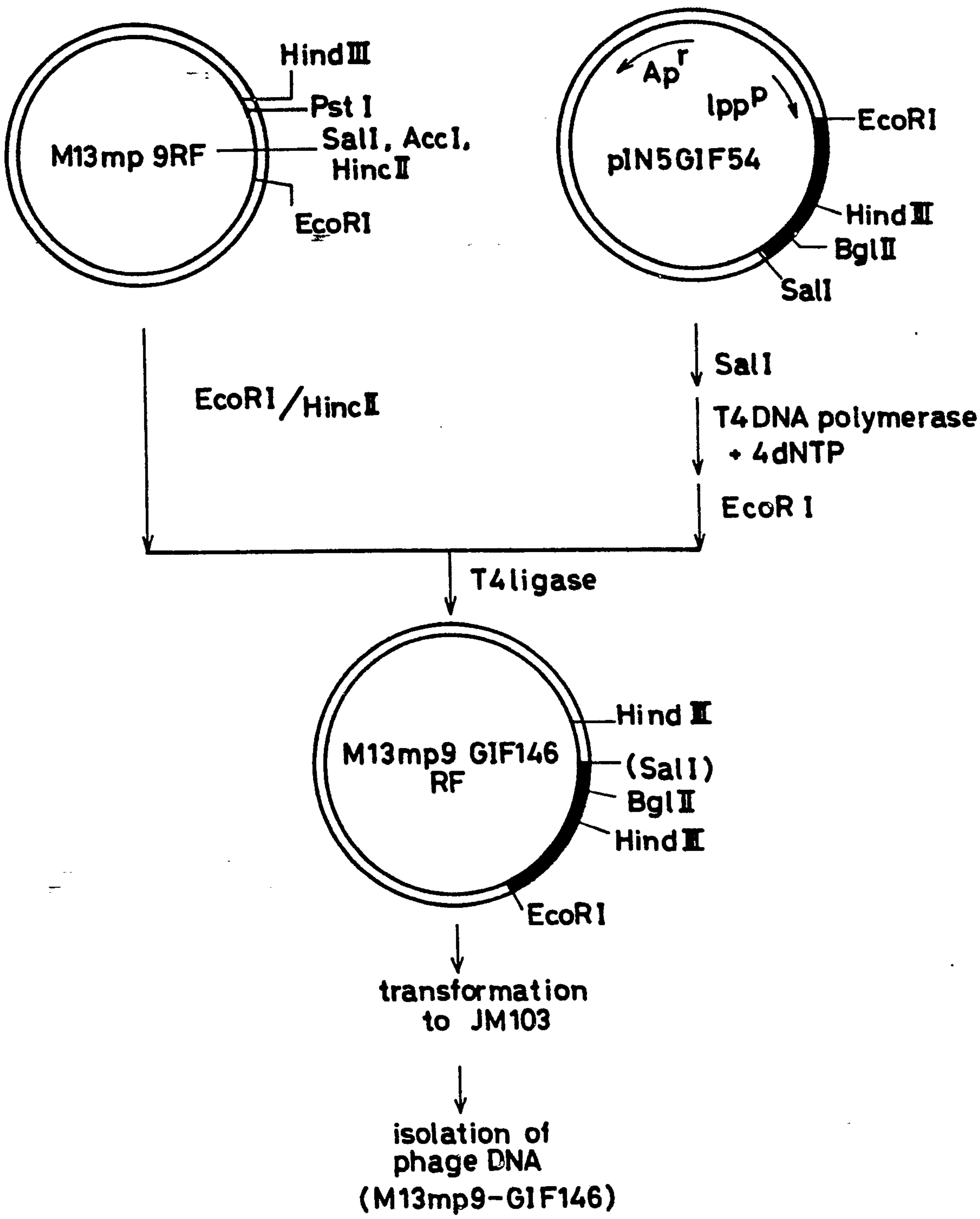

HindIII
Pst I
SalI, AccI,
HincII
M13mp 9RF
EcoRI
Ap^r
lpp^P
EcoRI
pIN5GIF54
HindIII
BglII
SalI
EcoRI/HincII
SalI
T4DNA polymerase
+ 4dNTP
EcoR I
T4ligase
Hind III
(SalI)
BglII
HindIII
M13mp9 GIF146
RF
EcoRI
transformation
to JM103
isolation of
phage DNA
(M13mp9-GIF146)

Fig. 5

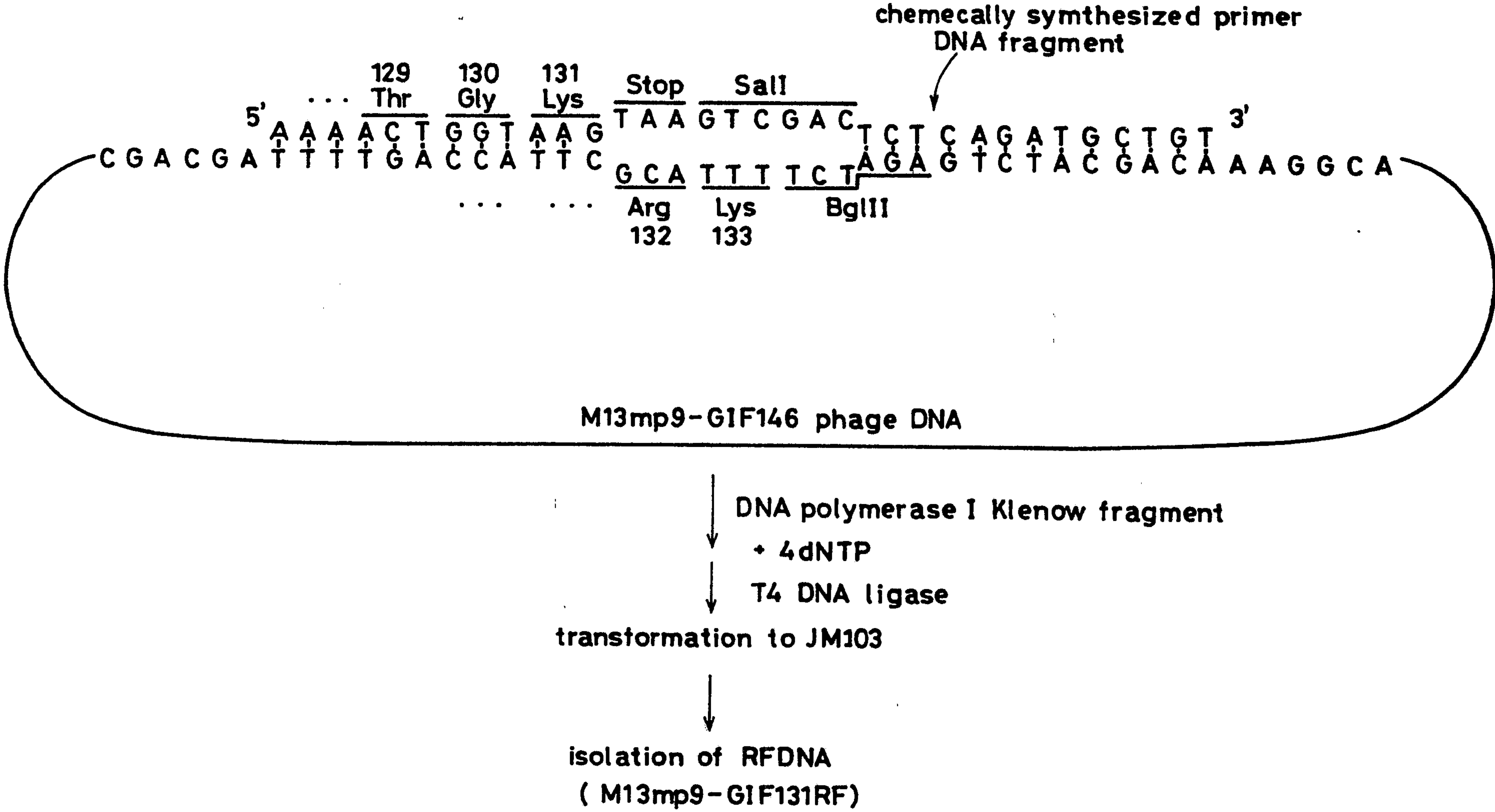

chemecally symthesized primer DNA fragment
5'
3'
129 Thr
130 Gly
131 Lys
Stop
SalI
Arg 132
Lys 133
BglII
AAAACTGGTAAGTAAGTCGACTCTCAGATGCTGT
CGACGATTTTGACCATTCGCATTTTCTAGAGTCTACGACAAAGGCA
M13mp9-GIF146 phage DNA
DNA polymerase I Klenow fragment
+ 4dNTP
T4 DNA ligase
transformation to JM103
isolation of RFDNA
( M13mp9-GIF131RF)

Fig. 6

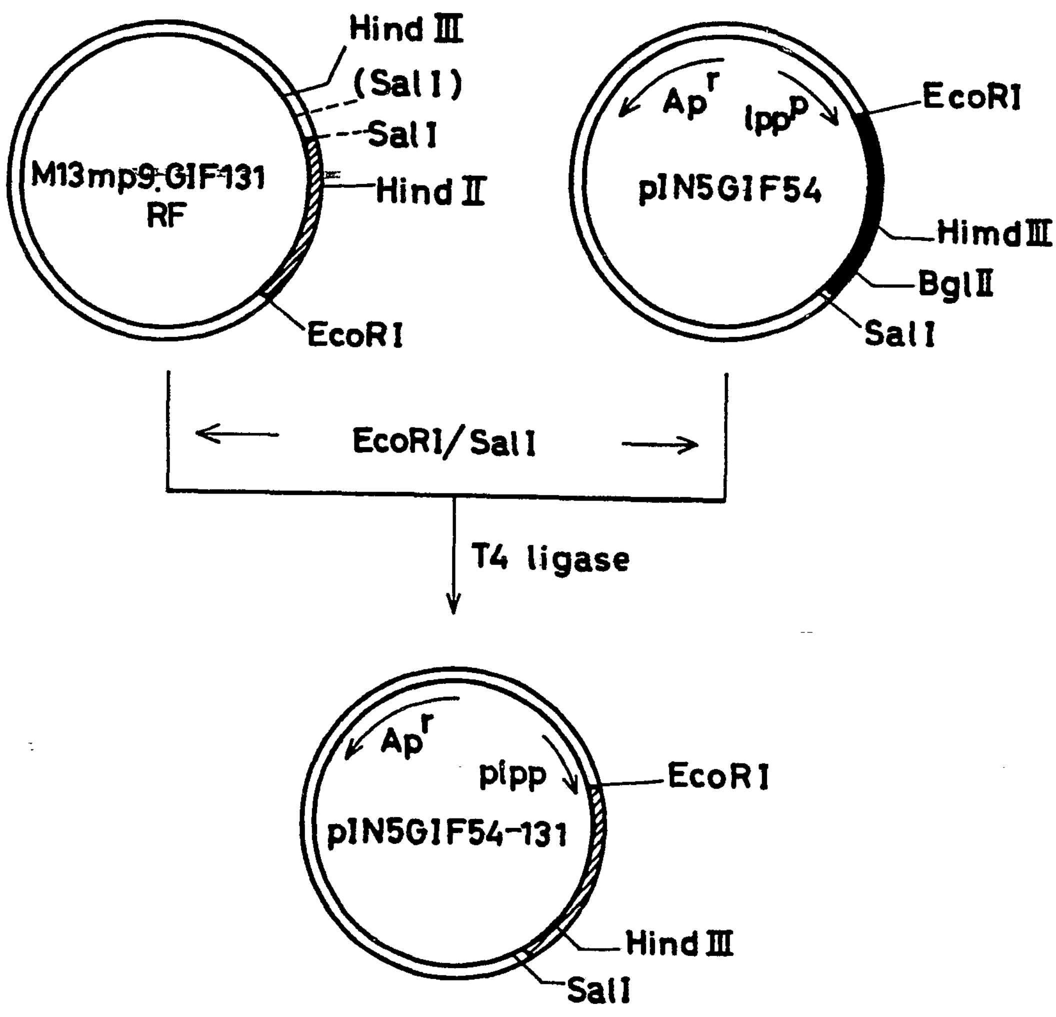
Hind III
(Sal I)
Sal I
M13mp9.GIF131
RF
Hind II
EcoRI
Ap^r
lpp^p
EcoRI
pIN5GIF54
HimdIII
BglII
SalI
EcoRI/SalI
T4 ligase
Ap^r
pIpp
EcoRI
pIN5GIF54-131
HindIII
SalI

Fig. 7

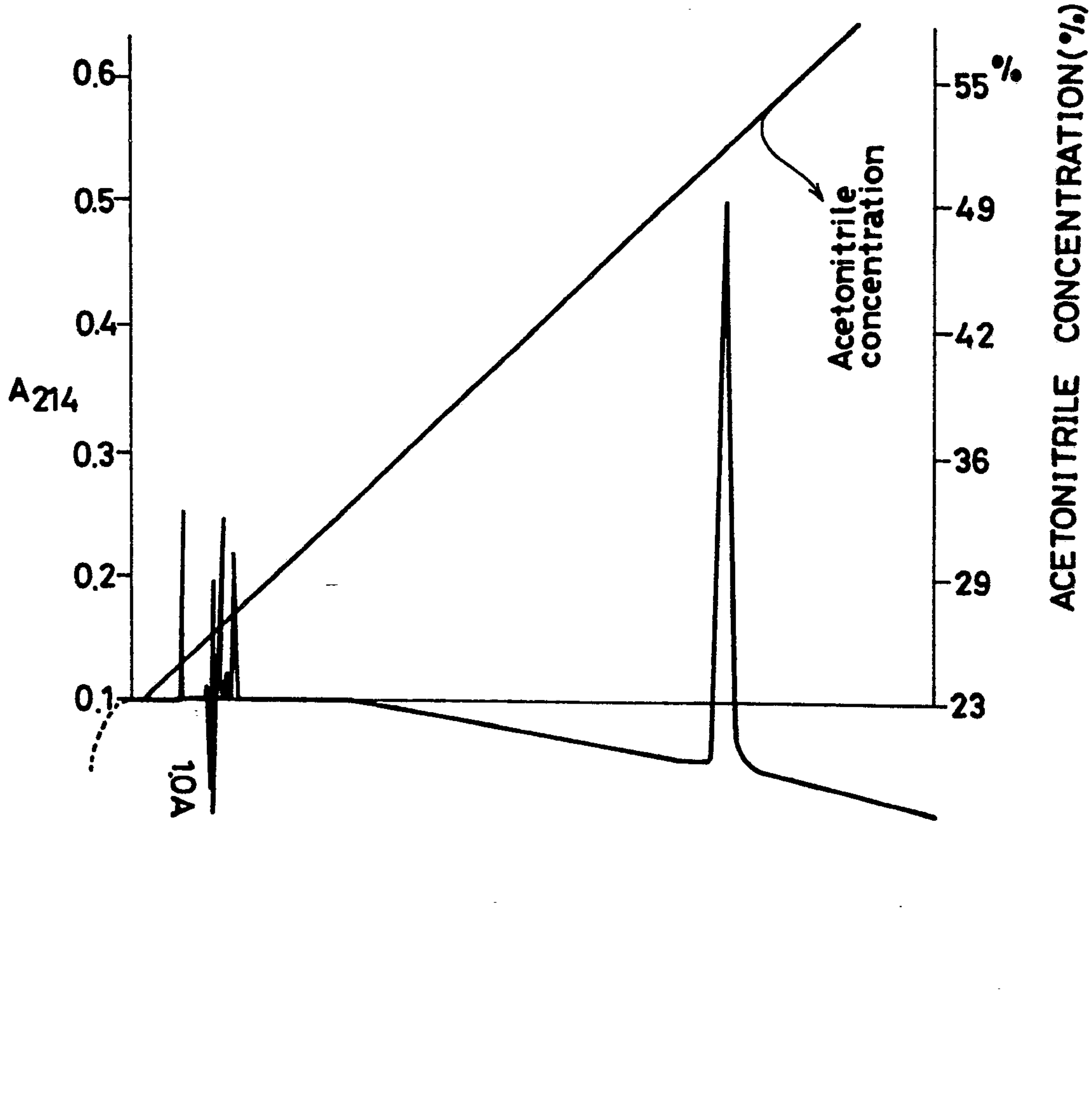
0.6
0.5
0.4
A214
0.3
0.2
0.1
10A
55 %
49
42
36
29
23
Acetonitrile concentration
ACETONITRILE CONCENTRATION (%)

0147175

8/15

Fig. 8

8-a

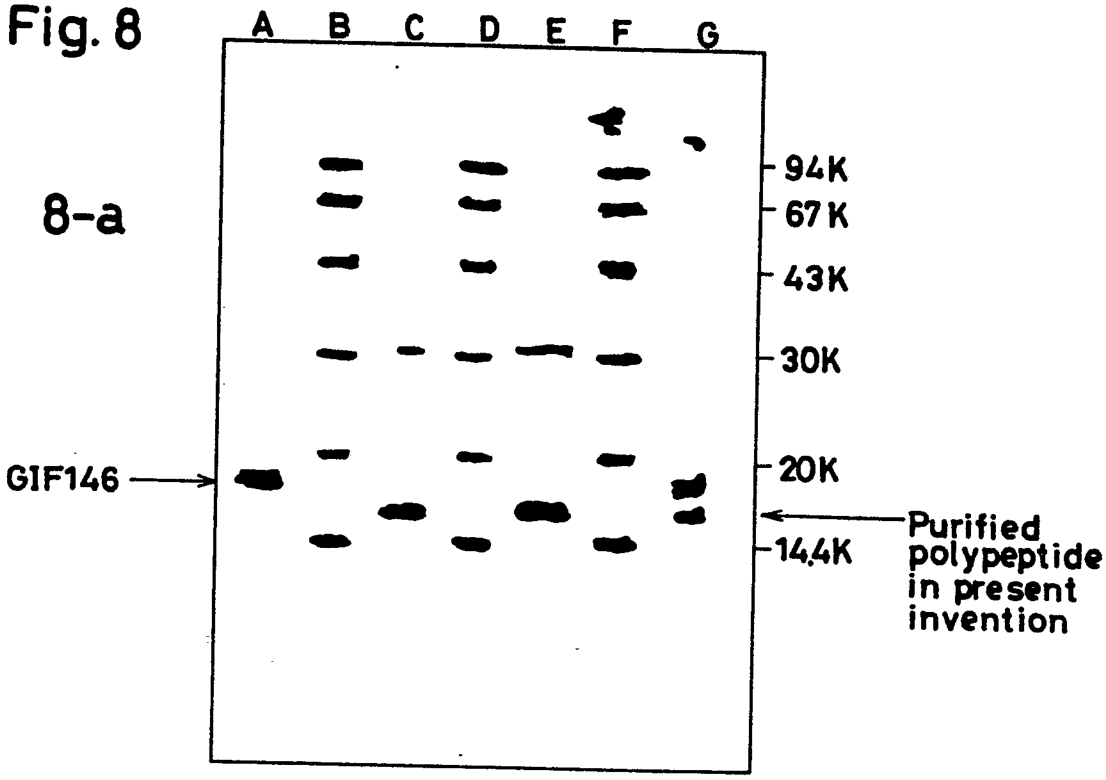


8-b

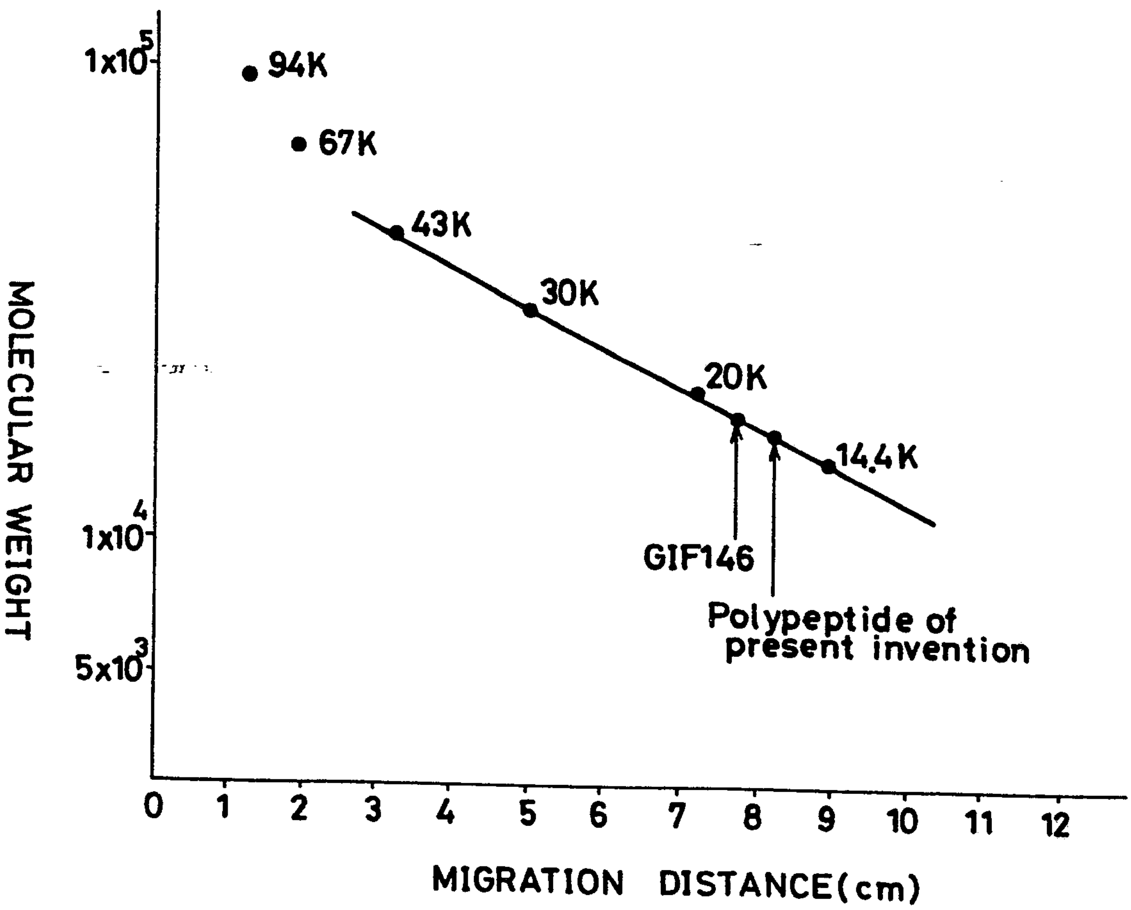

Fig. 9

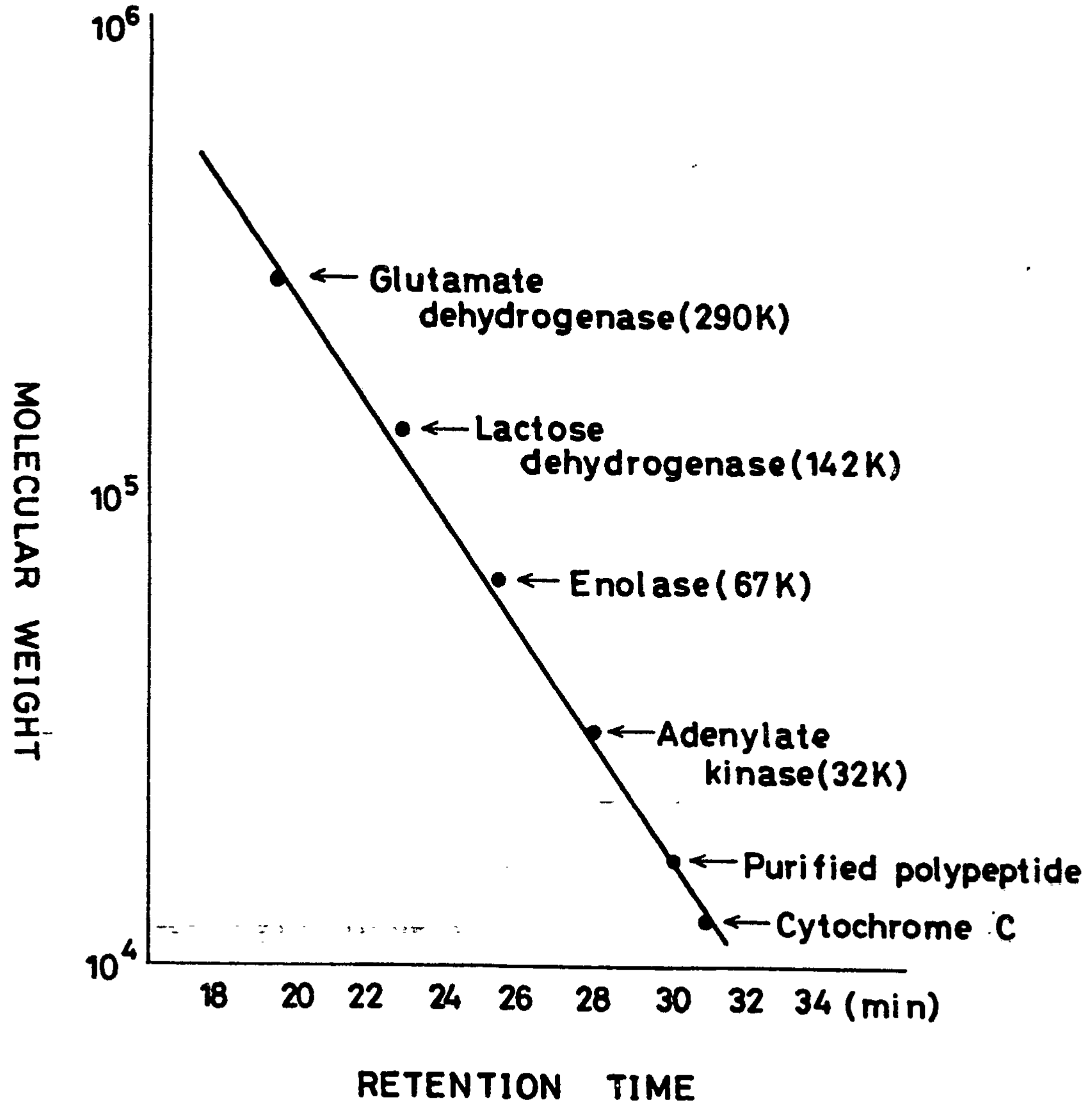
MOLECULAR WEIGHT
$10^6$
$10^5$
$10^4$
Glutamate dehydrogenase(290K)
Lactose dehydrogenase(142K)
Enolase(67K)
Adenylate kinase(32K)
Purified polypeptide
Cytochrome C
18
20
22
24
26
28
30
32
34 (min)
RETENTION TIME

Fig. 10

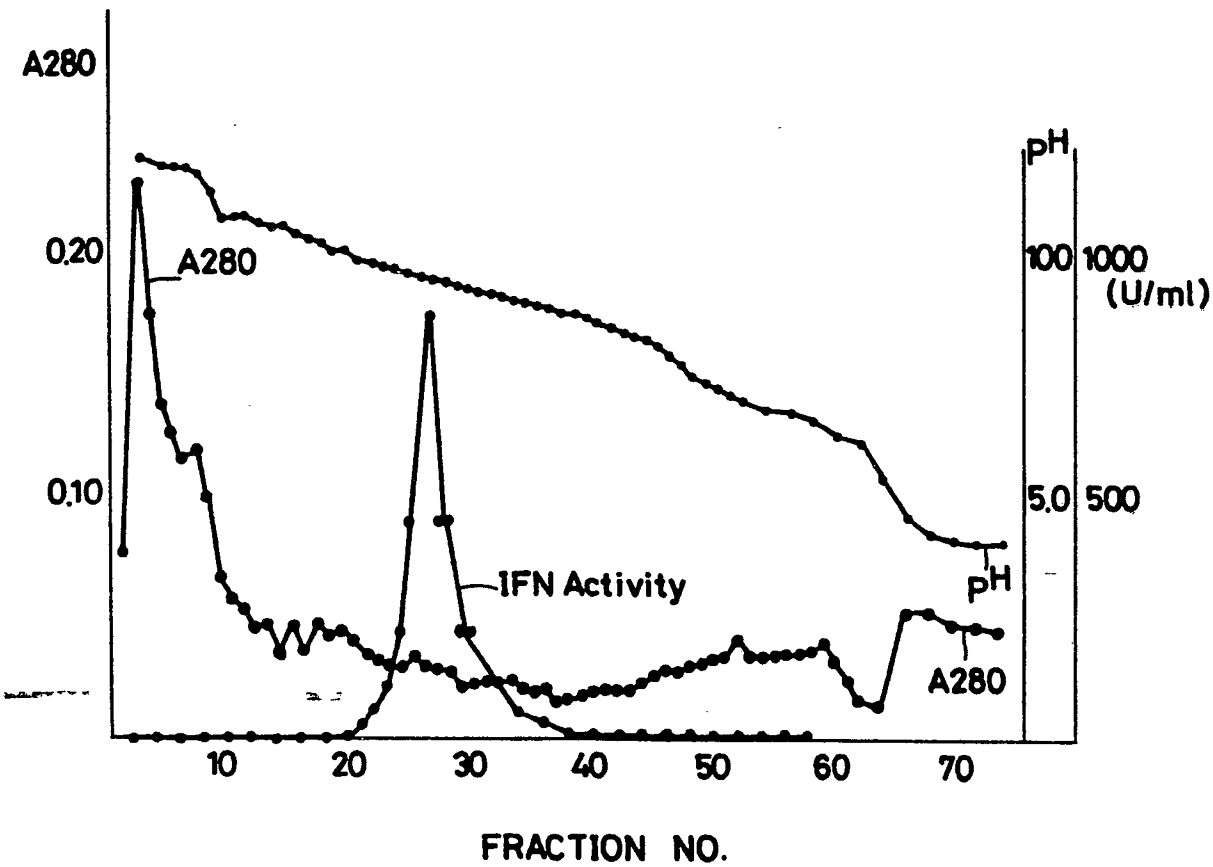
A280
0.20
0.10
A280
IFN Activity
pH
100
5.0
1000
(U/ml)
500
pH
A280
10
20
30
40
50
60
70
FRACTION NO.

# Fig. 11

1 Cys Tyr Cys Gln 5 Asp Pro Tyr Val Lys 10 Glu Ala Glu Asn Leu 15 Lys Lys Tyr Phe Asn 20 Ala Gly His Ser Asp 25 Val

Ala Asp Asn Gly 30 Thr Leu Phe Leu Gly 35 Ile Leu Lys Asn Trp 40 Lys Glu Glu Ser Asp 45 Arg Lys Ile Met Gln 50 Ser

Gln Ile Val Ser 55 Phe Tyr Phe Lys Leu 60 Phe Lys Asn Phe Lys 65 Asp Asp Gln Ser Ile 70 Gln Lys Ser Val Glu 75 Thr

I : BrCN (49-80)

Ile Lys Glu Asp 80 Met Asn Val Lys Phe 85 Phe Asn Ser Asn Lys 90 Lys Lys Arg Asp Asp 95 Phe Glu Lys Leu Thr 100 Asn

II : BrCN (81-120)

Tyr Ser Val Thr 105 Asp Leu Asn Val Gln 110 Arg Lys Ala Ile His 115 Glu Leu Ile Gln Val 120 Met Ala Glu Leu Ser 125 Pro

III : BrCN (121-131)

Ala Ala Lys Thr 130 Gly Lys

B4h (131)

Fig. 12

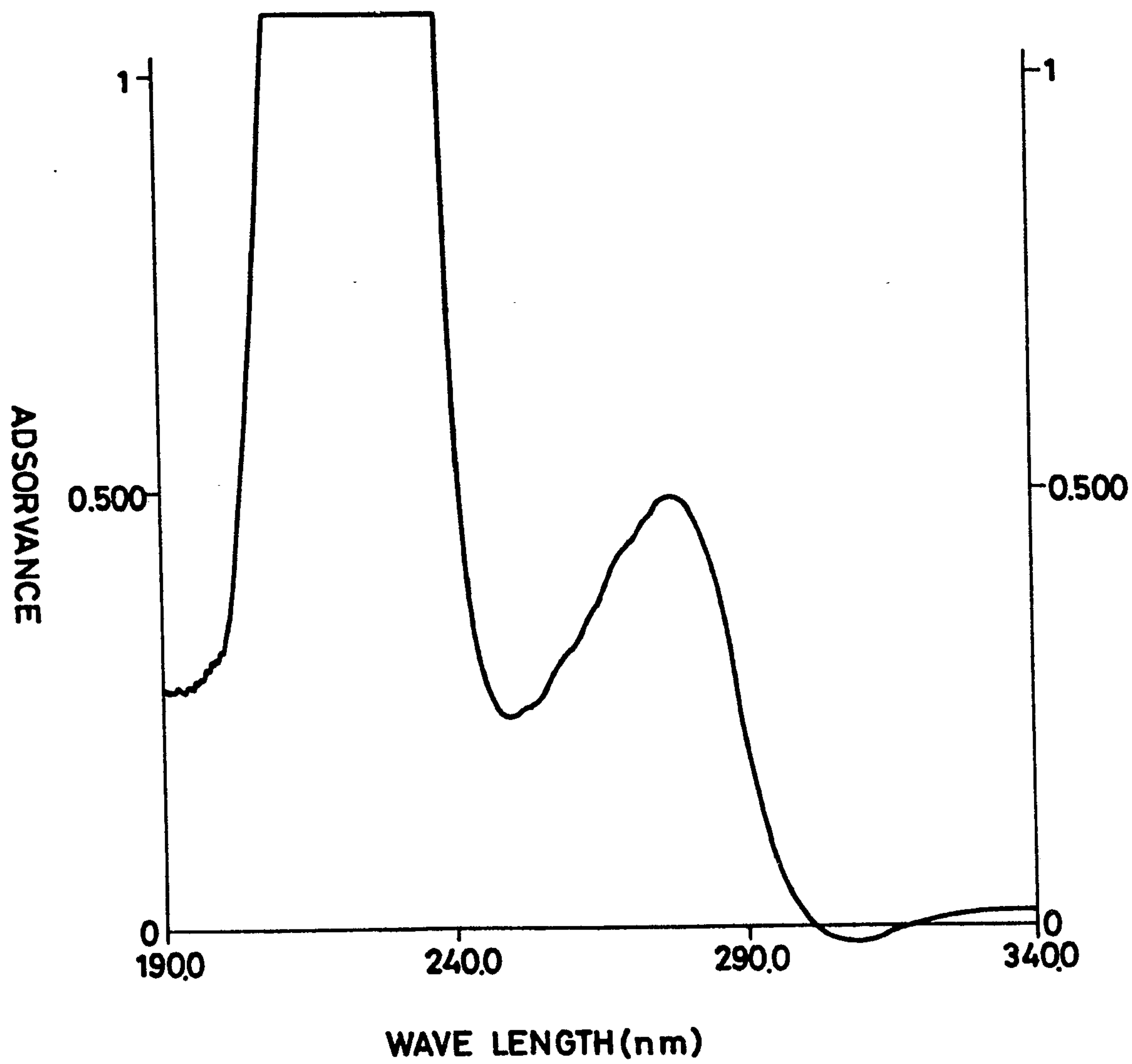

Fig. 13

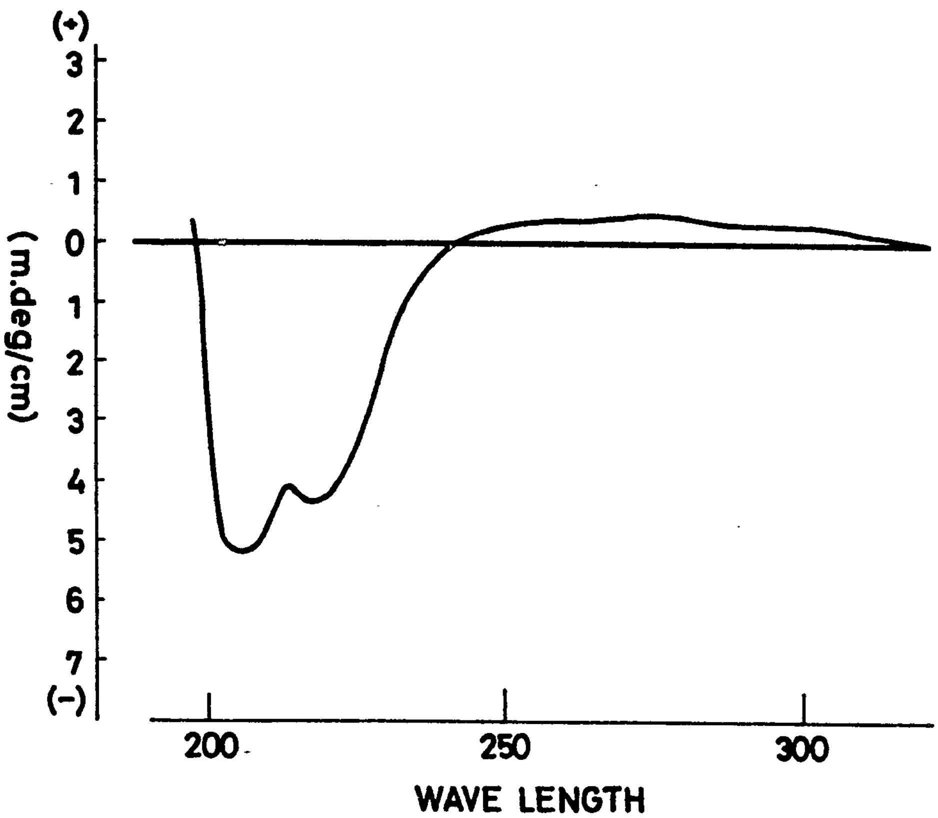

0147175

14/15

Fig. 14

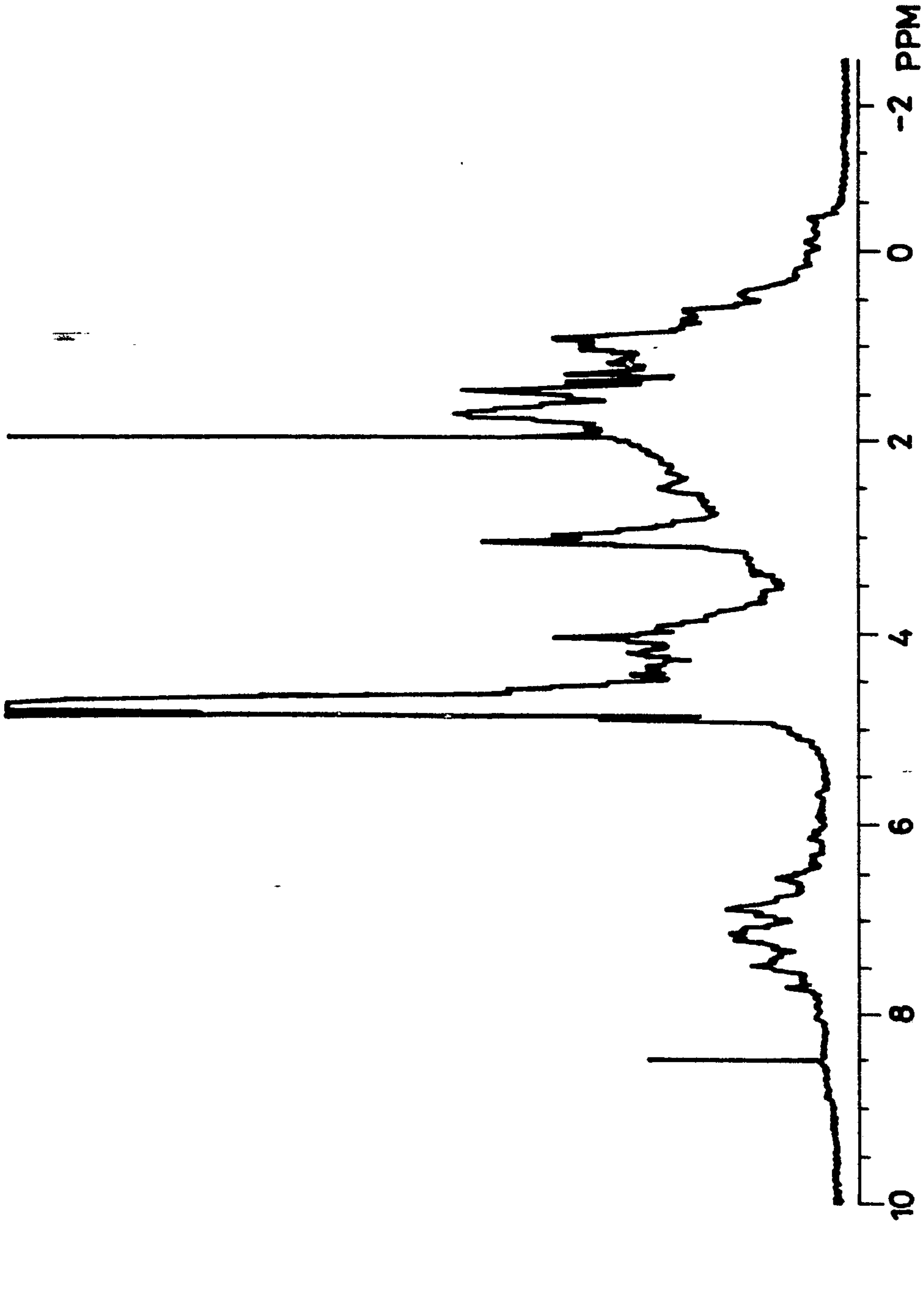

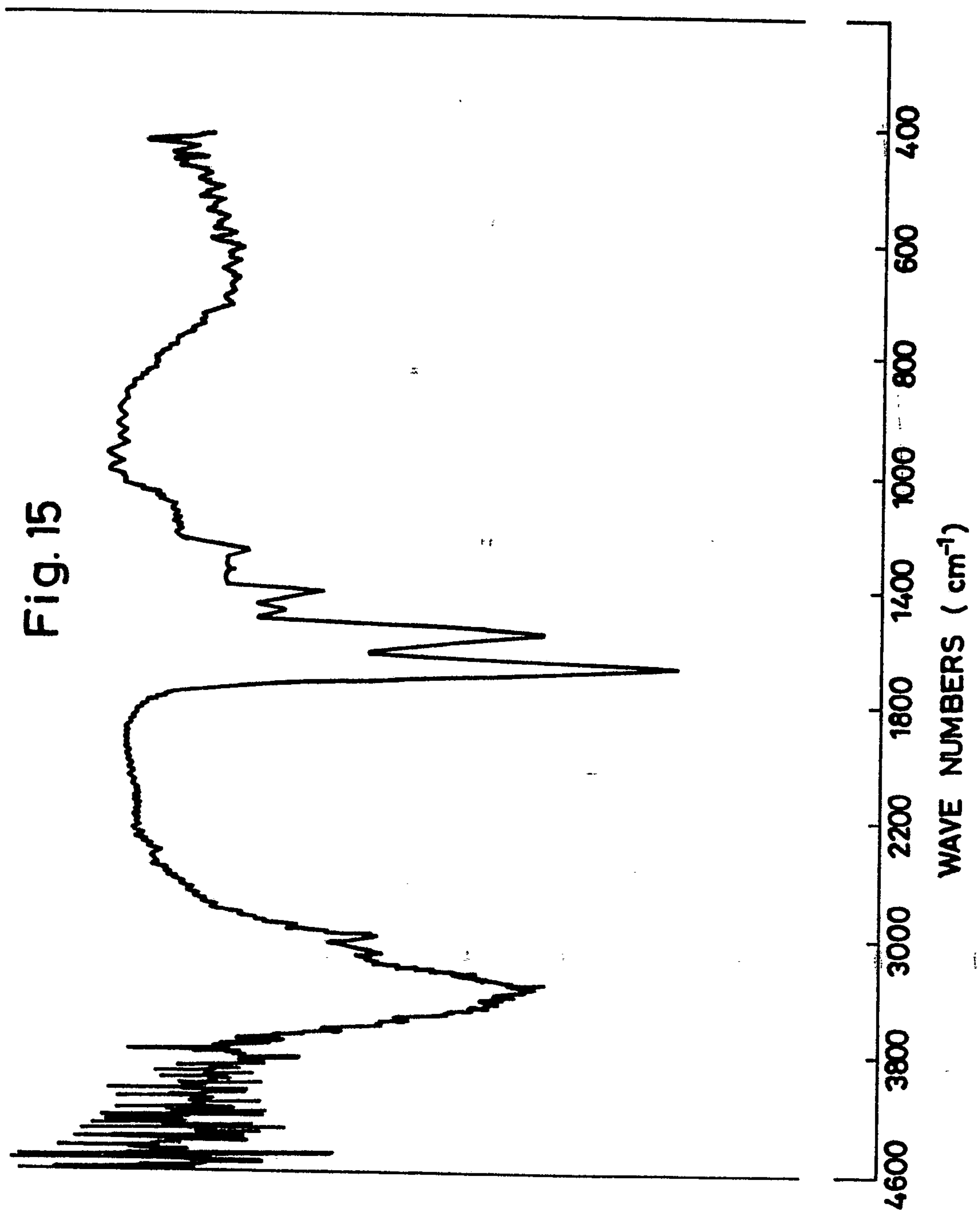
4600
3800
3000
2200
1800
1400
1000
800
600
400
WAVE NUMBERS ( cm-1)

Fig. 15